(19) Europäisches Patentamt
European Patent Office
Office européen des brevets

(11) **EP 4 025 127 B1**

(12) # EUROPEAN PATENT SPECIFICATION

(45) Date of publication and mention
of the grant of the patent:
**05.11.2025 Bulletin 2025/45**

(51) International Patent Classification (IPC):
*A61B 5/11* (2006.01)    *A61B 5/00* (2006.01)
*A61B 5/103* (2006.01)    *A63B 21/00* (2006.01)
*A63B 24/00* (2006.01)

(21) Application number: **20859835.9**

(22) Date of filing: **04.09.2020**

(52) Cooperative Patent Classification (CPC):
**A61B 5/112; A61B 5/1122; A61B 5/1126;**
A61B 5/6804; A61B 2505/09; A61B 2562/0219;
A61B 2562/04

(86) International application number:
**PCT/US2020/049509**

(87) International publication number:
**WO 2021/046423 (11.03.2021 Gazette 2021/10)**

(54) **QUANTIFICATION OF SYMMETRY AND REPEATABILITY IN LIMB MOTION FOR TREATMENT
OF ABNORMAL MOTION PATTERNS**

QUANTIFIZIERUNG VON SYMMETRIE UND WIEDERHOLBARKEIT IN EINER
SCHENKELBEWEGUNG ZUR BEHANDLUNG ABNORMALER BEWEGUNGSMUSTER

QUANTIFICATION DE SYMÉTRIE ET DE RÉPÉTABILITÉ DANS LE MOUVEMENT D'UN MEMBRE,
PERMETTANT LE TRAITEMENT DE MOTIFS DE MOUVEMENT ANORMAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.09.2019 US 201962896672 P**

(43) Date of publication of application:
**13.07.2022 Bulletin 2022/28**

(73) Proprietor: **The University of Miami
Miami, Florida 33136 (US)**

(72) Inventors:
• **GAILEY, Robert**
  **Miami, Florida 33136 (US)**
• **KIM, Kyoung Jae**
  **Miami, Florida 33136 (US)**

(74) Representative: **Hofstetter, Schurack & Partner
Patent- und Rechtsanwaltskanzlei
PartG mbB
Balanstraße 73 / Haus 31A
81541 München (DE)**

(56) References cited:
WO-A2-2004/099942    US-A1- 2013 324 888
US-A1- 2014 343 460    US-A1- 2016 074 272
US-A1- 2016 184 985    US-A1- 2017 273 601
US-A1- 2017 340 506    US-A1- 2019 150 793

• JARCHI DELARAM ET AL: "A Review on
Accelerometry-Based Gait Analysis and
Emerging Clinical Applications", IEEE REVIEWS
IN BIOMEDICAL ENGINEERING, vol. 11, 15
February 2018 (2018-02-15), pages 177 - 194,
XP011687581, ISSN: 1937-3333, [retrieved on
20180725], DOI: 10.1109/RBME.2018.2807182
• LI MENGXUAN ET AL: "Gait Analysis for Post-
Stroke Hemiparetic Patient by Multi-Features
Fusion Method", SENSORS, vol. 19, no. 7, 11
April 2019 (2019-04-11), CH, pages 1737,
XP093264809, ISSN: 1424-8220, DOI: 10.3390/
s19071737
• ANWARY ARIF REZA; YU HONGNIAN;
VASSALLO MICHAEL: "Gait Evaluation Using
Procrustes and Euclidean Distance Matrix
Analysis", IEEE JOURNAL OF BIOMEDICAL AND
HEALTH INFORMATICS, 9 November 2018
(2018-11-09), pages 2021 - 2029, XP011743835

**Description**

**CROSS-REFERENCE TO RELATED APPLICATIONS**

This application claims priority to U.S. Provisional Patent App. No. 62/896,672, filed on September 6, 2019.

**BACKGROUND**

Field of the Invention

The embodiments described herein are generally directed to treatment of abnormal motion patterns, and, more particularly, to the use of inertial sensors or other measurement devices to objectively quantify or otherwise measure symmetry and/or repeatability of motion in jointed limbs, such as a human subject's gait.

Description of the Related Art

Individuals with a lower limb amputation who use a prosthesis for ambulation commonly exhibit asymmetrical gait patterns. Those with more proximal levels of amputation (i.e., higher on the limb) often have greater gait asymmetries. Such gait asymmetries have been associated with increased risk for secondary health conditions, such as osteoarthritis, lower back pain, osteopenia, soft tissue damage, and balance impairments. Secondary effects, associated with lower limb amputation, can also impact these individuals' participation in activities and their quality of life. Therefore, the identification and subsequent treatment of gait asymmetries in individuals with a lower limb amputation can reduce long-term health effects associated with amputation, while improving mobility and overall well-being. However, traditional methods of gait assessment require expensive equipment, such as computerized gait laboratories or instrumented surfaces, as well as significant space, technical expertise, and time.

In addition, document US 2013/ 074 272 A1 describes a method and apparatus for recognizing a gait task, document US 2013/ 324 333 A1 describes a system and method for measuring balance and track motion in mammals, and document US 2016/ 184 985 A1 describes an assisting torque setting method and apparatus.

**SUMMARY**

The invention is defined by the appended claims. Accordingly, systems, methods, and non-transitory computer-readable media are disclosed for measuring symmetry and repeatability in jointed limbs for treatment of abnormal (e.g., asymmetrical) motion patterns. While embodiments will generally be described herein with respect to data collected by inertial sensors for measuring symmetry and repeatability in gait patterns of the lower limbs (i.e., subjects' intact and/or prosthetic limbs), it should be understood that the processes described herein may be used with data collected by other means (e.g., video-based motion capture) and/or to measure symmetry and repeatability in motion patterns for other jointed limbs (e.g., subjects' intact and/or injured, post-surgical, and/or pathological, lower or upper limbs).

In an embodiment, a method is disclosed that comprises using at least one hardware processor to: acquire gait data comprising a signal from each of a plurality of inertial sensors, wherein each signal represents an angular velocity of one of a plurality of lower limb segments of a subject during ambulation; segment each signal into a plurality of stride signals, wherein each of the plurality of stride signals represents one of a plurality of strides during the ambulation; calculate at least one gait metric based on the pluralities of stride signals, wherein the at least one gait metric comprises one or both of a gait symmetry metric that represents a similarity of the plurality of stride signals across two of the signals acquired for at least one pair of contralateral ones of the plurality of lower limb segments, and a gait repeatability metric that represents a similarity of the plurality of stride signals within at least one of the signals to each other; and output the at least one gait metric. Furthermore, the at least one gait metric comprises the gait symmetry metric, wherein calculating the gait symmetry metric comprises aligning contralateral pairs of the plurality of stride signals across the two signals acquired for the at least one pair of contralateral lower limb segments. In addition, calculating the gait symmetry metric further comprises calculating a distance between each aligned contralateral pair of stride signals; and calculating a mean of the calculated distances; and/or the distance is a Euclidean distance; and/or the gait symmetry metric is calculated as:

$$100 - \left( 100 \times \frac{mean\ of\ the\ calculated\ distances}{threshold_S} \right)$$

wherein $threshold_S$ is a threshold representing an estimated maximum possible mean of the calculated distances.

The signal may be acquired for each of the plurality of lower limb segments of the subject during ambulation, wherein the plurality of lower limb segments comprises a right thigh, right shank, left thigh, and left shank of the subject. Each signal may represent the angular velocity in a sagittal, coronal, and/or transverse plane of the subject over a time period of the ambulation. Acquiring the gait data may comprise receiving a wireless signal transmitted by one or more inertial measurement units, positioned on the plurality of lower limb segments of the subject, wherein the one or more inertial measurement units comprise the plurality of inertial sensors. Each of the plurality of stride signals may represent one of a plurality of strides from a toe-off to a next toe-off.

The ambulation may comprise one or more ambulation tests. The one or more ambulation tests may comprise a distance-based walk test. The gait data may consist of signals from the plurality of inertial sensors collected during a middle portion of the distance-based walk test.

Aligning the contralateral pairs of stride signals may comprise dynamic time warping.

The at least one gait metric may comprise the gait repeatability metric, wherein calculating the gait repeatability metric comprises aligning the plurality of stride signals within the at least one signal. Aligning the plurality of stride signals within the at least one signal may comprise dynamic time warping. Calculating the gait repeatability metric may further comprise: averaging the aligned plurality of stride signals within the at least one signal into an average stride signal; calculating a distance between each of the plurality of stride signals within the at least one signal and the average stride signal; and calculating a mean of the calculated distances. The distance may be a Euclidean distance. The gait repeatability metric may be calculated as:

$$100 - \left(100 \times \frac{mean\ of\ the\ calculated\ distances}{threshold_R}\right)$$

wherein $threshold_R$ is a threshold representing an estimated maximum possible mean of the calculated distances.

Any of the methods may be embodied in executable software modules of a processor-based system, such as a server, and/or in executable instructions stored in a non-transitory computer-readable medium.

## BRIEF DESCRIPTION OF THE DRAWINGS

The details of the present disclosure, both as to its structure and operation, may be gleaned in part by study of the accompanying drawings, in which like reference numerals refer to like parts, and in which:

FIG. 1 illustrates an example processing system, by which one or more of the processes described herein, may be executed, according to an embodiment;
FIG. 2 illustrates an example sensor system, which may be used to acquire gait data, according to an embodiment;
FIG. 3 illustrates the sagittal plane relative to a subject;
FIG. 4 illustrates an example process for providing a symmetry metric, according to an embodiment;
FIG. 5 illustrates an example process for providing a repeatability metric, according to an embodiment; and
FIG. 6 illustrates an intermediate representation of the calculation of a gait repeatability metric, according to an embodiment.

## DETAILED DESCRIPTION

The invention is defined by the appended claims.

Systems, methods, and non-transitory computer-readable media are disclosed for measuring symmetry and/or repeatability (e.g., in gait), using a measurement device (e.g., inertial sensors), for treatment of abnormal motion (e.g., gait) patterns. In an embodiment, body-worn inertial measurement units (IMUs) are used to collect large amounts of data regarding movement of limb segments of one or more jointed limbs in one or a plurality of planes. For example, for lower limbs, these limb segments may comprise a subject's intact thigh, prosthetic thigh, intact shank, and/or prosthetic shank. For upper limbs, these limb segments may comprise a subject's intact upper arm, prosthetic upper arm, intact lower arm or forearm, and prosthetic lower arm or forearm. In general, it should be understood that a jointed limb is any limb comprising at least two segments joined by a joint (e.g., knee or elbow), regardless of whether those segments are intact or prosthetic. For example, since an IMU will operate the same regardless of whether it is positioned on an intact segment or a prosthetic segment, it should be understood that, as used herein, the term "thigh" may refer to an intact thigh or prosthetic thigh, and the term "shank" may refer to an intact shank or prosthetic shank. In addition, it should be understood that any of the disclosed systems and processes that apply to a prosthetic limb segment can also be applied, without minimal or no modification, to any limb segment that does not have complete function, such as an injured, post-surgical, and/or pathological limb segment.

Embodiments will primarily be described herein with respect to the measurement of gait patterns in the lower limbs of a subject. For example, subjects may be instructed to perform predefined functional tasks with his or her lower limbs (e.g., walking, standing, sitting, ascending and/or descending ramps or stairs, etc.). Subjects with unilateral lower limb pathology will likely exhibit asymmetry between lower limb segments. Thus, it can be beneficial to independently examine each lower limb segment (i.e., contralateral thighs and contralateral shanks). Understanding where the differences occur within ipsilateral lower limb segments or between contralateral lower limb segments can assist with objective differential diagnosis and clinical treatment prescription. Notably, the contribution of individual lower limb segments, as measured by IMUs, to specific gait parameters and ambulatory performance has not been previously explored.

However, it should be understood that the disclosed embodiments may be used, with or without modification, for the measurement of motion patterns in the upper limbs of a subject or in the collective motion patterns of both the lower limbs and the upper limbs of a subject. In this case, the subject may be instructed to perform predefined functional tasks with his or her upper limbs, such as repetitively performing one or more motions (e.g., reaching with the arms, bending at the elbow, rotating the wrist, etc.). The symmetry in motion between contralateral upper limb segments and the repeatability of motion for an ipsilateral upper limb segment can be measured in the same way as for the lower limb segments. The data are processed in the same manner, regardless of whether the data was collected from lower limb segments or upper limb segments. In other words, the disclosed processes are limb agnostic, but are primarily described with respect to the lower limbs in order to convey the benefits and advantages of one application of the disclosed processes.

## 1. System Overview

### 1.1. Example Processing System

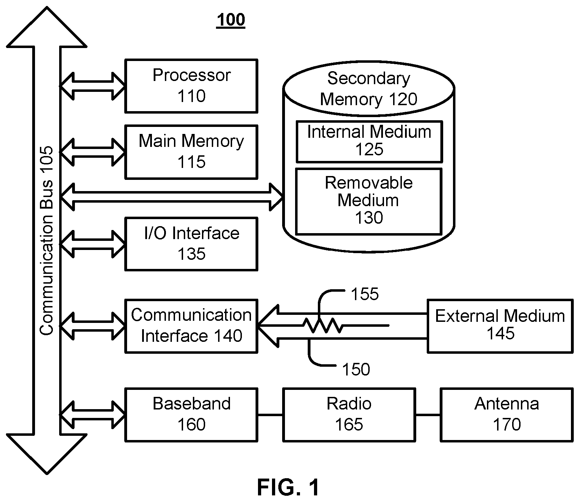

FIG. 1 is a block diagram illustrating an example processing system 100 that may be used in connection with various embodiments described herein, including in conjunction with one or more of the functions, processes, or methods described herein. For example, processing system 100 may store and/or execute one or more software modules that implement one or more of the processes described herein. Software module(s) that implement one or any combination of the discloses processes will be collectively referred to herein as "the application."

Processing system 100 can be a server, a conventional personal computer (e.g., desktop computer, laptop computer, etc.), a mobile device (e.g., smartphone, tablet computer, etc.), a measurement unit (e.g., an IMU or system for processing signals from a plurality of IMUs), or any other processor-enabled device. Processing system 100 may be configured for wired and/or wireless data communication. A person of skill in the art will understand that computer systems and/or architectures, other than those illustrated and described herein, may be also used. Furthermore, one or a plurality of the components described with respect to processing system 100 may be omitted in various embodiments or devices.

Processing system 100 preferably includes one or more processors, such as processor 110. Additional processors may be provided, such as an auxiliary processor to manage input/output, an auxiliary processor to perform floating-point mathematical operations, a special-purpose microprocessor having an architecture suitable for fast execution of signalprocessing algorithms (e.g., digital-signal processor), a slave processor subordinate to the main processing system (e.g., back-end processor), an additional microprocessor or controller for dual or multiple processor systems, and/or a coprocessor. Such auxiliary processors may be discrete processors or may be integrated with processor 110. Examples of processors which may be used with system 100 include, without limitation, the Pentium® processor, Core i7® processor, and Xeon® processor, all of which are available from Intel Corporation of Santa Clara, California.

Processor 110 is preferably connected to a communication bus 105. Communication bus 105 may include a data channel for facilitating information transfer between storage and other peripheral components of processing system 100. Furthermore, communication bus 105 may provide a set of signals used for communication with processor 110, including a data bus, address bus, and/or control bus (not shown). Communication bus 105 may comprise any standard or non-standard bus architecture such as, for example, bus architectures compliant with industry standard architecture (ISA), extended industry standard architecture (EISA), Micro Channel Architecture (MCA), peripheral component interconnect (PCI) local bus, standards promulgated by the Institute of Electrical and Electronics Engineers (IEEE) including IEEE 488 general-purpose interface bus (GPIB), IEEE 696/S-100, and/or the like.

Processing system 100 preferably includes a main memory 115 and may also include a secondary memory 120. Main memory 115 provides storage of instructions and data for programs executing on processor 110, such as the application discussed herein. It should be understood that programs stored in main memory 115 and executed by processor 110 may be written and/or compiled according to any suitable language, including without limitation C/C++, Java, JavaScript, Perl, Visual Basic, .NET, and the like. Main memory 115 is typically semiconductor-based memory such as dynamic random access memory (DRAM) and/or static random access memory (SRAM). Other semiconductor-based memory types include, for example, synchronous dynamic random access memory (SDRAM), Rambus dynamic random access memory (RDRAM), ferroelectric random access memory (FRAM), and the like, including read only memory (ROM).

Secondary memory 120 may optionally include an internal medium 125 and/or a removable medium 130. Removable medium 130 is read from and/or written to in any well-known manner. Removable storage medium 130 may be, for example, a magnetic tape drive, a compact disc (CD) drive, a digital versatile disc (DVD) drive, other optical drive, a flash memory drive, and/or the like. Secondary memory 120 is a non-transitory computer-readable medium having computer-executable code (e.g., the disclosed application) and/or other data stored thereon. The computer software or data stored on secondary memory 120 is read into main memory 115 for execution by processor 110.

In an alternative embodiment, secondary memory 120 may include other similar means for allowing computer programs or other data or instructions to be loaded into processing system 100. Such means may include, for example, a communication interface 140, which allows software and data to be transferred from external storage medium 145 to processing system 100. Examples of external storage medium 145 may include an external hard disk drive, an external optical drive, an external magneto-optical drive, and/or the like. Other examples of secondary memory 120 may include semiconductor-based memory, such as programmable read-only memory (PROM), erasable programmable read-only memory (EPROM), electrically erasable read-only memory (EEPROM), and flash memory (block-oriented memory similar to EEPROM).

As mentioned above, processing system 100 may include a communication interface 140. Communication interface 140 allows software and data to be transferred between system 100 and external devices (e.g. printers), networks, or other information sources. For example, computer software or executable code may be transferred to processing system 100 from a network server via communication interface 140. Examples of communication interface 140 include a built-in network adapter, network interface card (NIC), Personal Computer Memory Card International Association (PCMCIA) network card, card bus network adapter, wireless network adapter, Universal Serial Bus (USB) network adapter, modem, a wireless data card, a communications port, an infrared interface, an IEEE 1394 fire-wire, and any other device capable of interfacing system 100 with a network or another computing device. Communication interface 140 preferably implements industry-promulgated protocol standards, such as Ethernet IEEE 802 standards, Fiber Channel, digital subscriber line (DSL), asynchronous digital subscriber line (ADSL), frame relay, asynchronous transfer mode (ATM), integrated digital services network (ISDN), personal communications services (PCS), transmission control protocol/Internet protocol (TCP/IP), serial line Internet protocol/point to point protocol (SLIP/PPP), and so on, but may also implement customized or non-standard interface protocols as well.

Software and data transferred via communication interface 140 are generally in the form of electrical communication signals 155. These signals 155 may be provided to communication interface 140 via a communication channel 150. In an embodiment, communication channel 150 may be a wired or wireless network, or any variety of other communication links. Communication channel 150 carries signals 155 and can be implemented using a variety of wired or wireless communication means including wire or cable, fiber optics, conventional phone line, cellular phone link, wireless data communication link, radio frequency ("RF") link, or infrared link, just to name a few.

Computer-executable code (e.g., the disclosed application) is stored in main memory 115 and/or secondary memory 120. Computer programs can also be received via communication interface 140 and stored in main memory 115 and/or secondary memory 120. Such computer programs, when executed, enable processing system 100 to perform the various functions of the disclosed embodiments as described elsewhere herein.

In this description, the term "computer-readable medium" is used to refer to any non-transitory computer-readable storage media used to provide computer-executable code and/or other data to or within processing system 100. Examples of such media include main memory 115, secondary memory 120 (including internal memory 125, removable medium 130, and external storage medium 145), and any peripheral device communicatively coupled with communication interface 140 (including a network information server or other network device). These non-transitory computer-readable media are means for providing executable code, programming instructions, software, and/or other data to processing system 100.

In an embodiment that is implemented using software, the software may be stored on a computer-readable medium and loaded into processing system 100 by way of removable medium 130, I/O interface 135, or communication interface 140. In such an embodiment, the software is loaded into processing system 100 in the form of electrical communication signals 155. The software, when executed by processor 110, preferably causes processor 110 to perform one or more of the processes and functions described elsewhere herein.

In an embodiment, I/O interface 135 provides an interface between one or more components of processing system 100 and one or more input and/or output devices. Example input devices include, without limitation, sensors, keyboards, touch screens or other touch-sensitive devices, biometric sensing devices, computer mice, trackballs, pen-based pointing devices, and/or the like. Examples of output devices include, without limitation, other processing devices, cathode ray tubes (CRTs), plasma displays, light-emitting diode (LED) displays, liquid crystal displays (LCDs), printers, vacuum fluorescent displays (VFDs), surface-conduction electron-emitter displays (SEDs), field emission displays (FEDs), and/or the like. In some cases, an input and output device may be combined, such as in the case of a touch panel display (e.g., in a smartphone, tablet computer, or similar mobile device).

Processing system 100 may also include optional wireless communication components that facilitate wireless communication over a voice network and/or a data network (e.g., in the case of a smartphone). The wireless communication components comprise an antenna system 170, a radio system 165, and a baseband system 160. In processing system 100, radio frequency (RF) signals are transmitted and received over the air by antenna system 170 under the management of radio system 165.

In an embodiment, antenna system 170 may comprise one or more antennae and one or more multiplexors (not shown) that perform a switching function to provide antenna system 170 with transmit and receive signal paths. In the receive path, received RF signals can be coupled from a multiplexor to a low noise amplifier (not shown) that amplifies the received RF signal and sends the amplified signal to radio system 165.

In an alternative embodiment, radio system 165 may comprise one or more radios that are configured to communicate over various frequencies. In an embodiment, radio system 165 may combine a demodulator (not shown) and modulator (not shown) in one integrated circuit (IC). The demodulator and modulator can also be separate components. In the incoming path, the demodulator strips away the RF carrier signal leaving a baseband receive audio signal, which is sent from radio system 165 to baseband system 160.

If the received signal contains audio information, then baseband system 160 decodes the signal and converts it to an analog signal. Then the signal is amplified and sent to a speaker. Baseband system 160 also receives analog audio signals from a microphone. These analog audio signals are converted to digital signals and encoded by baseband system 160. Baseband system 160 also encodes the digital signals for transmission and generates a baseband transmit audio signal that is routed to the modulator portion of radio system 165. The modulator mixes the baseband transmit audio signal with an RF carrier signal, generating an RF transmit signal that is routed to antenna system 170 and may pass through a power amplifier (not shown). The power amplifier amplifies the RF transmit signal and routes it to antenna system 170, where the signal is switched to the antenna port for transmission.

Baseband system 160 is also communicatively coupled with processor 110, which may be a central processing unit (CPU). Processor 110 has access to data storage areas 115 and 120. Processor 110 is preferably configured to execute instructions (e.g., the disclosed application) that can be stored in main memory 115 or secondary memory 120. The instructions can also be received from baseband processor 160 and stored in main memory 110 or in secondary memory 120, or executed upon receipt. The disclosed application, when executed by processing system 100, enables processing system 100 to perform one or any combination of the disclosed processes.

1.2. <u>Sensor System</u>

In an embodiment, a sensor system of one or more body-worn inertial sensors is used to collect gait data which are used by the disclosed application to assess a subject's gait symmetry and/or repeatability. For example, the system may comprise a plurality (e.g., four) inertial measurement units (IMUs) that each comprise at least one inertial sensor. IMUs are generally small, inexpensive, wireless devices that can be worn on the body, are not restricted by time and space, and can provide innumerable amounts of data regarding human motion without requiring the installation of bulky, heavy, expensive equipment. This makes IMUs especially useful for a point-of-care device. A typical IMU comprises two or more inertial sensors, including an accelerometer and a gyroscope, and, in some cases, a magnetometer. In an embodiment, each IMU comprises at least a gyroscope that continuously outputs a gyroscopic signal.

The IMUs may be arranged within the system, such that, when the system is worn on the lower limbs of a subject, a first IMU is positioned on the subject's right thigh, a second IMU is positioned on the subject's right shank, a third IMU is positioned on the subject's left thigh, and a fourth IMU is positioned on the subject's left shank. In this case, the first and third IMUs may be positioned on a distal-lateral area of the respective thigh, and the second and fourth IMUs may be positioned on the proximal-medial area of the respective shank. When the system is worn on the upper limbs of a subject, a first IMU may be positioned on the subject's right upper arm, a second IMU may be positioned on the subject's right forearm, a third IMU may be positioned on the subject's left upper arm, and a fourth IMU may be positioned on the subject's left forearm. In this case, the first and third IMUs may be positioned on a distal lateral area of the respective upper arm, and the second and fourth IMUs may be positioned on the proximal-medial area of the respective forearm.

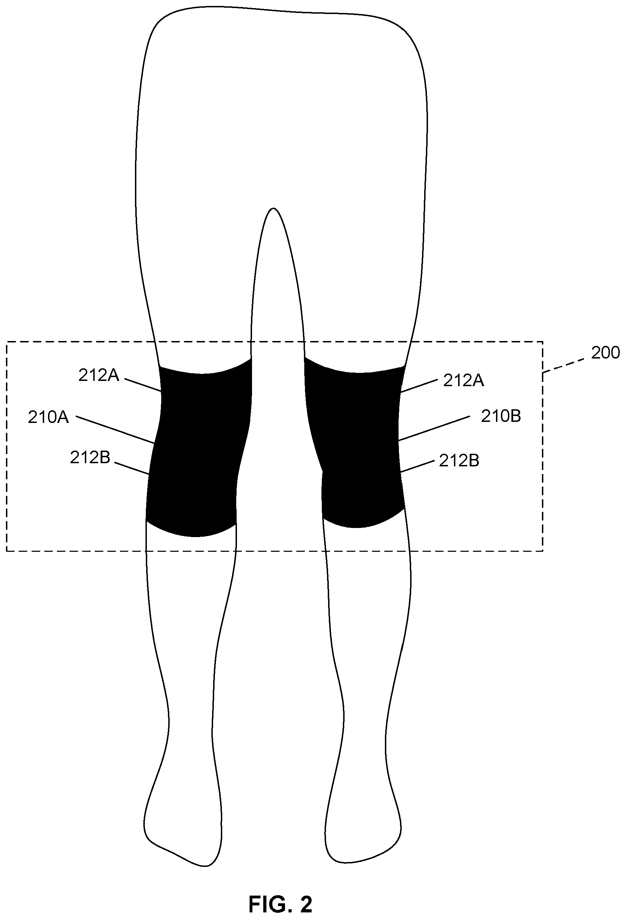

FIG. 2 illustrates a sensor system 200 that may be used to collect the motion (e.g., gait) data, according to an embodiment. Sensor system 200 may comprise two sleeves 210, illustrated as sleeve 210A and 210B. Each sleeve 210 comprises one or more IMUs 212. Each IMU 212 comprises an inertial sensor that outputs a gyroscopic signal. IMUs 212 may be attached to sleeve 210 in any standard manner. For example, each IMU 212 may be sewn into sleeve 210, adhered, taped, sewn, or otherwise fastened to an outside or inside of sleeve 210, magnetically attached to an outside or inside of sleeve 210, and/or the like. Each IMU 212 may be permanently attached to sleeve 210, or detachably attached to sleeve 210 (e.g., for replacement, recharging, establishing a wired connection, etc.).

In the illustrated embodiment, each sleeve 210 comprises two IMUs 212A and 212B attached to opposite sides and ends of sleeve 210. However, each sleeve 210 could consist of a different number of IMUs 212, including one IMU 212, three IMUs 212, four IMUs 212 (e.g., on both the lateral and medial sides of each limb above and below the knee or elbow), and so on and so forth.

Each IMU 212 may further comprise a battery that powers the inertial sensor. Alternatively, each sleeve 210 may comprise a single battery that powers all IMUs 212 (e.g., IMUs 212A and 212B) on that sleeve 210. For example, the battery may supply power to every IMU 212 on a sleeve via electrically conductive traces or wires sewn into sleeve 210, adhered, taped, sewn, or otherwise fastened to an outside or inside of sleeve 210, and/or the like.

Each IMU 212 may also comprise a communication interface 140 for wired communication and/or wireless communication (e.g., via baseband system 160, radio 165, and antenna 170) of the motion data collected by the inertial sensor. Alternatively, each sleeve 210 may comprise a single communication interface 140 that collects the motion data from all IMUs 212 on the sleeve 210 via wires sewn into sleeve 210, adhered, taped, sewn, or otherwise fastened to an outside or inside of sleeve 210, and/or the like. In either case, communication interface 140 may be configured to transmit the motion data collected by the inertial sensor(s) via wired and/or wireless communication to an external system (e.g., server, desktop computer, mobile device such as a smartphone or tablet computer, etc.). Thus, gyroscopic signals from all of the IMUs 212 can be recorded at the external system for use by one or more of the processes described elsewhere herein.

In addition, each IMU 212 or each sleeve 210 may comprise a memory (e.g., main memory 115 and/or secondary 120) that stores the motion data prior to communication. For example, in an embodiment which implements wireless communication, motion data may be stored in the memory and automatically wirelessly transmitted to the external system periodically (e.g., after each expiration of a fixed time interval, as soon as a certain amount of motion data has been stored, etc.). In an embodiment that does not implement wireless communication, all of the motion data may be stored in the memory until it is downloaded to the external system via a wired connection (e.g., via a connection to a Universal Serial Bus (USB) socket in sleeve 210 or in each IMU 212). To prioritize memory usage, the memory may store a rolling window of motion data in which the oldest data are continually overwritten by the newest data. Each IMU 212 or each sleeve 210 could also comprise a processor 110 that preprocesses the motion data prior to storage in the memory, after storage in the memory, and/or before communication to the external system.

It should be understood that sleeves 210A and 210B may be identical. However, in order for motion data to be collected from corresponding sides of the limb, sleeve 210B may be rotated 180 degrees with respect to sleeve 210A or turned upside-down with respect to sleeve 210A, so that IMUs 212 on both sleeves 210 are on corresponding sides of the respective limb to ensure that the motion data are consistently measured across limbs. As illustrated, the upper IMUs 212A of both sleeves 210A and 210B are positioned on the lateral side of the thigh, whereas the lower IMUs 212B of both sleeves 210A and 210B are positioned on the medial side of the shank. In alternative embodiments, all IMUs 212 of both sleeves 210A and 210B may be positioned on the lateral side of the limb, all IMUs 212 of both sleeves 210A and 210B may be positioned on the medial side of the limb, or the upper IMUs 212A may be positioned on the medial side of the limb while the lower IMUs 212B are positioned on the lateral side of the limb.

Sleeve 210 may be made from flexible material (e.g., stretchable fabric, rubber, etc.) or within an orthosis (e.g., a joint brace composed of plastic, composite materials, carbon fiber, etc., to form a smart knee brace or smart elbow brace), in order to accommodate limbs of different sizes. In addition, the length of sleeve 210 and/or the positioning of IMUs 212A and 212B on sleeve 210 may be chosen so as to, when sleeve 210 is worn, generally position a first IMU 212A near a distal end of the upper limb segment (e.g., thigh or upper arm) of the subject (i.e., proximate to and above the knee or elbow) and a second IMU 212B near a proximal end of the lower limb segment (e.g., shank or forearm) of the subject (i.e., proximate to and below the knee or elbow). Again, IMUs 212 may be positioned on the lateral and/or medial side of the limb. However, in a preferred embodiment, each sleeve 210 is configured to position a first IMU 212A on the distal-lateral thigh or upper arm and a second IMU 212B on the proximal-medial shank or forearm, as illustrated in FIG. 2 with respect to the lower limbs.

While sensor system 200 is primarily described herein as comprising two sleeves 210A and 210B, sensor system 200 could alternatively consist of a single sleeve 210. In such a case, the motion data for a single limb may be used for one or more of the disclosed processes. Alternatively, the motion data from one limb may be acquired by the single sleeve 210 first, and then the single sleeve 210 may be moved to the other limb to acquire the motion data for that other limb. In this case, the motion data from both limbs may subsequently be combined for use by one or more of the disclosed processes.

In addition, while sensor system 200 will primarily be described in the context of collecting motion data for a subject with a unilateral limb amputation (e.g., unilateral lower limb amputation or unilateral upper limb amputation), sensor system 200 could be used with a subject having bilateral limb amputations (e.g., bilateral lower limb amputation or bilateral upper limb amputation) and/or no limb amputation (e.g., otherwise healthy subjects, subjects with orthopedic or neurological injuries or pathologies, subjects recovering from surgery, etc.). Indeed, sensor system 200 could be used to collect motion data for any subject who may benefit from a measurement of limb symmetry and/or repeatability, such as gait symmetry and/or repeatability.

In an alternative embodiment, sensor system 200 could comprise separate sleeves or bands for each IMU 212. Each band may encircle a single limb segment to position one or more IMUs 212 on that limb segment. In this case, each band may be identical to each other, and any number of bands could be combined to form a sensor system 200 with any number of IMUs 212 and any arrangement of IMUs 212 (e.g., with one band on each limb segment, multiple bands on each limb segment, one band on one limb segment and multiple bands on a different limb segment, etc.).

In yet another embodiment, sensor system 200 may omit sleeves altogether. In this case, IMU(s) 212 and/or individual inertial sensor(s) (e.g., a gyroscope) may be positioned on a subject using other means, such as by direct adherence to the subject (e.g., using adhesive, such as double-sided tape between the IMU 212 and limb segment, one-sided tape or other wrap wrapped around the IMU 212 and limb segment, etc.), being sewn into or otherwise attached to a garment (e.g., pants or shirt) worn by the subject, and/or the like.

In another alternative embodiment, sensor system 200 may comprise sensors other than IMUs 212 and/or sensors that are not worn by the subject. For example, sensor system 200 could comprise a camera-based motion capture system that captures video or still images of a subject performing a repetitive movement test, and analyzes the captured image frames to compute motion data, such as the sagittal angular velocity of one or more limb segments of the subject.

2. Process Overview

Embodiments of processes for measuring motion symmetry and/or repeatability (e.g., of a subject's gait) - for example, using inertial sensors - for treatment of abnormal motion patterns (e.g., gait patterns) will now be described in detail. It should be understood that the described processes may be embodied in one or more software modules that are executed by one or more hardware processors 110 as the application discussed herein. The described processes may be implemented as instructions represented in source code, object code, and/or machine code. These instructions may be executed directly by the hardware processor(s) 110, or alternatively, may be executed by a virtual machine operating between the object code and hardware processors 110. In addition, the disclosed application may be built upon or interfaced with one or more existing systems.

Alternatively, the described processes may be implemented as a hardware component (e.g., general-purpose processor, integrated circuit (IC), application-specific integrated circuit (ASIC), digital signal processor (DSP), field-programmable gate array (FPGA) or other programmable logic device, discrete gate or transistor logic, etc.), combination of hardware components, or combination of hardware and software components. To clearly illustrate the interchangeability of hardware and software, various illustrative components, blocks, modules, circuits, and steps are described herein generally in terms of their functionality. Whether such functionality is implemented as hardware or software depends upon the particular application and design constraints imposed on the overall system. Skilled persons can implement the described functionality in varying ways for each particular application, but such implementation decisions should not be interpreted as causing a departure from the scope of the invention. In addition, the grouping of functions within a component, block, module, circuit, or step is for ease of description. Specific functions or steps can be moved from one component, block, module, circuit, or step to another without departing from the invention.

Furthermore, while the processes, described herein, are illustrated with a certain arrangement and ordering of subprocesses, each process may be implemented with fewer, more, or different subprocesses and a different arrangement and/or ordering of subprocesses. In addition, it should be understood that any subprocess, which does not depend on the completion of another subprocess, may be executed before, after, or in parallel with that other independent subprocess, even if the subprocesses are described or illustrated in a particular order.

2.1. Data Collection

In an embodiment, motion data are collected using sensor system 200 to detect and quantify the movement of four segments of a subject's jointed limbs (e.g., bilateral thighs and shanks, or bilateral upper arms and forearms) during one or more motion tests (e.g., ambulation tests). For example, when collecting gait data as the motion data, the subject may wear sleeves 210A and 210B on both lower limbs, such that IMUs 212A are positioned on the distal-lateral thighs of the subject and IMUs 212B are positioned on the proximal-medial shanks of the subject. As the subject performs one or more ambulation tests, output signals of the inertial sensors of all of the IMUs 212 are collected as the gait data. In alternative embodiments, the gait data may be collected from fewer lower limb segments (e.g., just bilateral thighs, just bilateral shanks, etc.), more lower limb segments, or different lower limb segments (e.g., knee, ankle, foot, etc.).

This motion data can be processed by an external system (e.g., processing system 100, which may be a desktop computer, tablet computer, smartphone, etc.) to compute the subject's motion symmetry and/or repeatability. Alternatively, sensor system 200 could compute the subject's motion symmetry and/or repeatability onboard, for example, using a processing system 100 embedded in sleeve 210. In either case, the computed motion symmetry and/or repeatability may provide insight into the subject's motion pattern (e.g., with a lower limb prosthesis, upper limb prosthesis, etc.).

In an embodiment in which a gait pattern is being measured, the subject may perform one or more ambulation tests. The one or more ambulation tests may comprise a distance-based walk test, such as a ten-meter walk test. For example, a subject may perform one or more trials of walking ten meters at a self-selected walking speed. The initial two meters and the final two meters may be used as acceleration and deceleration zones, respectively, while data collected during the middle six meters is used as the gait data. In the event that multiple trials of the ten-meter walk test are performed, the gait data may be averaged over all of the trials.

Alternatively or additionally, the ambulation test(s) may comprise other types of walk tests and/or other variations of a distance-based walk test (e.g., different distances). Other types of walk tests include, without limitation, a time-based walk test in which a subject walks for a particular amount of time (e.g., two minutes), an environment-based walk test in which a subject walks in a plurality of different environments (e.g., different terrains, such as dirt, grass, concrete, etc., and/or across different features, such as ramps, curbs, stairs, etc.), and/or the like.

Alternatively or additionally, the ambulation test(s) may comprise other types of ambulation. Other types of ambulation include, without limitation, running, jogging, speed walking, and/or the like. As with the walk tests, these other types of ambulation may be tested using distance-based, time-based, and/or environment-based tests.

In an embodiment in which a motion pattern of upper limbs is being measured, the subject may perform one or more repetitive motions. For example, these repetitive motions may comprise reaching out the arms, pulling in the arms, and/or performing some other motion that requires bending the arms at the elbow. Additionally or alternatively, these repetitive motions may comprise rotating the wrist, rotating the forearm, rotating the arm at the shoulders, and/or the like.

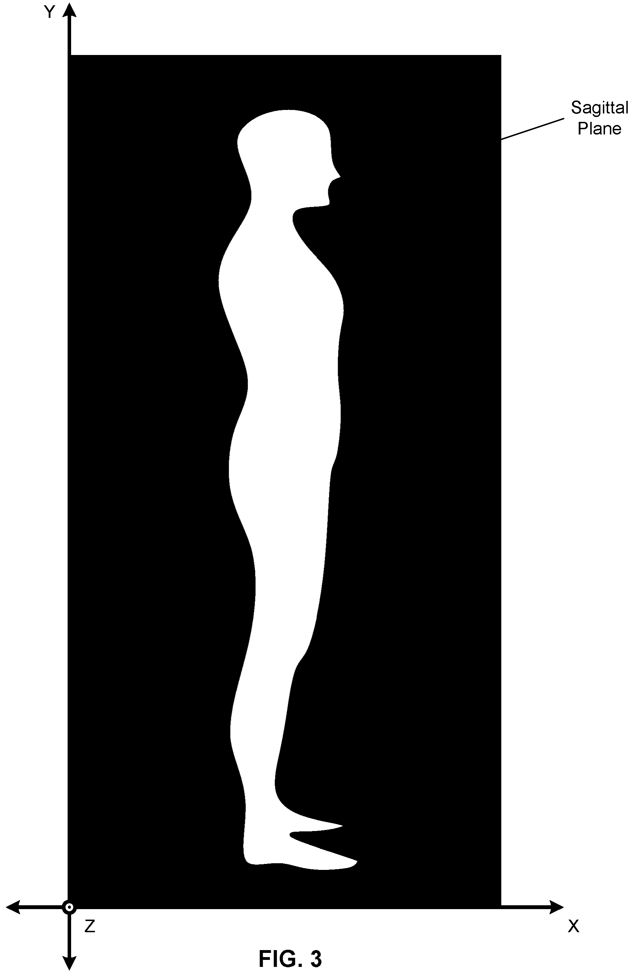

In an embodiment, the motion data comprise raw angular (rotational) velocities of the inertial sensors in the sagittal plane. As illustrated in FIG. 3, the well-known sagittal plane extends along an axis X through the posterior and anterior (rear and front) of a subject and along an axis Y through the superior and inferior (top and bottom) of the subject. The sagittal plane is orthogonal to axis Z, which extends orthogonally through the viewing plane of FIG. 3. As will be understood by a person of ordinary skill, the sagittal plane is also orthogonal to the coronal plane (defined by axes Y and Z) and the transverse plane (defined by axes X and Z). In an alternative embodiment, the motion data may comprise raw angular velocities in the coronal and/or transverse planes in combination or instead of the sagittal plane. However, the processes will be primarily described herein as using the sagittal angular velocities (i.e., angular velocities in the sagittal plane).

In a preferred embodiment, IMUs 212 of sensor system 200 wirelessly transmit their respective angular velocity signals to an external processing system 100. The angular velocities may be continually transmitted at periodic intervals (e.g., 60 Hz), so as to be acquired as a signal by the external processing system 100. The external processing system 100 may then process the acquired signals to perform the disclosed analysis and compute the disclosed symmetry and/or repeatability metrics.

## 2.2. Motion Analysis

In an embodiment, the motion data (e.g., gait data), collected from a subject, are analyzed to compute one or both of: a segmental symmetry score (SSS) which measures symmetry (i.e., contralateral symmetry); and a segmental repeatability score (SRS) which measures repeatability (i.e., ipsilateral symmetry). More specifically, SSS measures the similarity of angular velocities (e.g., sagittal angular velocities) of a limb segment and its corresponding contralateral limb segment between contralateral movements (e.g., strides during ambulation). In other words, SSS measures the symmetry of motion between contralateral limb segments. SRS, on the other hand, measures the similarity of angular velocities (e.g., sagittal angular velocities) of the same limb segment between consecutive movements (e.g., strides during ambulation). In other words, SRS measures the repeatability of motion by the same limb segment. In the context of gait analysis, SSS is a measure of the similarity between strides of contralateral lower limb segments, and SRS is a measure of the similarity between strides of the same (i.e., ipsilateral) lower limb segment.

In an embodiment, the motion data are collected using at least four IMUs placed above and below the joint on each of two contralateral limbs. For example, in the context of gait analysis, a first IMU 212 (e.g., IMU 212A of sleeve 210A) may be placed on the distal-lateral right thigh, a second IMU 212 (e.g., IMU 212B of sleeve 210A) may be placed on the proximal-medial right shank, a third IMU 212 (e.g., IMU 212A of sleeve 210B) may be placed on the distal-lateral left thigh, and a fourth IMU 212 (e.g., IMU 212B of sleeve 210B) may be placed on the proximal-medial left shank. In the context of arm-motion analysis, a first IMU 212 (e.g., IMU 212A of sleeve 210A) may be placed on the distal-lateral right upper arm, a second IMU 212 (e.g., IMU 212B of sleeve 210A) may be placed on the proximal-medial right forearm, a third IMU 212 (e.g., IMU 212A of sleeve 210B) may be placed on the distal-lateral left upper arm, and a fourth IMU 212 (e.g., IMU 212B of sleeve 210B) may be placed on the proximal-medial left forearm. Each IMU 212 continually captures the angular velocities (e.g., at a rate of 60 Hz), at its respective position on its respective limb segment, for example, during a motion test (e.g., ambulation test).

In an embodiment, the captured angular velocities are processed and interpreted through a dynamic time warping (DTW) algorithm to precisely quantify the movement differences of the limb segments through each motion segment (e.g., stride) during at least a portion of the motion test (e.g., the middle six meters of a ten-meter walk test). Examples of DTW algorithms that may be used are described in "Stride segmentation during free walk movements using multi-dimensional subsequence dynamic time warping on inertial sensor data," by Barth et al., Sensors, 2015, 15(3):6419-40, "Exposure to an extreme environment comes at a sensorimotor cost," by Kim et al., NPJ Microgravity, 2018, 4:1-8, and "Using inertial sensors to quantify balance and gait performance during the tandem walking test," by Kim et al., Sensors, 2019, 19(4). DTW enables the identification of motion segments (e.g., strides) with different lengths by matching signals non-linearly, such that subparts of a motion segment (e.g., stride) get stretched or shortened by warping a reference signal upon a target signal to achieve an optimal fit between the reference and target signals. In other words, DTW aligns segmented motion segments (e.g., strides) with each other so that they can be directly compared, despite differences in length.

Specific algorithms for calculating SSS and SRS will now be described. It should be understood that the described algorithms represent specific embodiments, and that SSS and/or SRS may be calculated differently in alternative embodiments. In addition, in an embodiment, both SSS and SRS may be calculated. In this case, for the sake of efficiency, certain subprocesses, such as data collection and/or motion segmentation, may be performed only once, with the same output used by both the SSS and SRS algorithms. In an alternative embodiment, only SSS or only SRS may be calculated.

The calculations of SSS and SRS will be described with respect to sagittal angular velocities. However, it should be understood that sensor system 200 (e.g., inertial sensors of IMUs 212) may output angular velocity signals for all three of the sagittal, coronal, and transverse planes. Thus, any one or any combination of these angular velocities may be used in calculating SSS and/or SRS. For example, in an alternative embodiment, the sagittal angular velocities described herein may be replaced with coronal angular velocities, transverse angular velocities, or any aggregation of sagittal, coronal, and/or transverse angular velocities.

2.2.1. Symmetry (SSS)

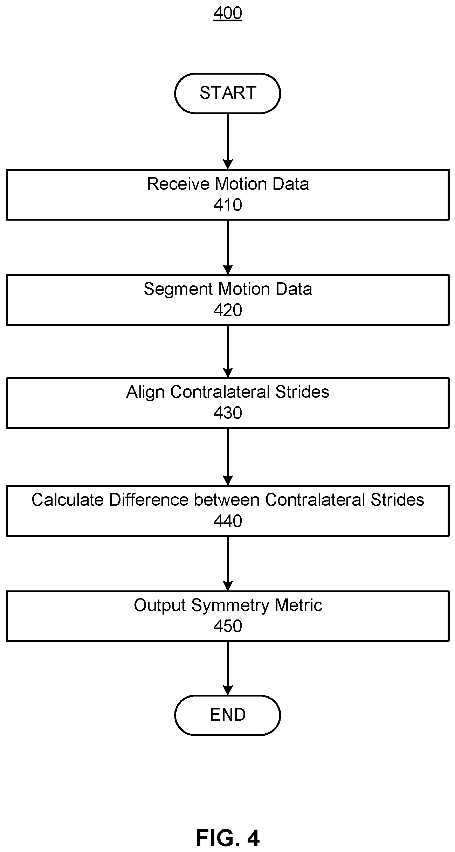

FIG. 4 illustrates a process 400 for calculating a symmetry metric, such as SSS, according to an embodiment. Process 400 may be implemented by the disclosed application, which may be executed by an external processing system 100 that is remote from sensor system 200 and receives motion data, directly or indirectly, from sensor system 200 via wireless or wired communication. Alternatively, the application may be executed by an onboard processing system 100 that is local to sensor system 200.

In subprocess 410, motion data (e.g., gait data) are received for one or more subjects. The motion data may be collected using sensor system 200, as described elsewhere herein. In an embodiment, the motion data for each subject comprise angular velocities, measured by IMUs 212, in the sagittal plane of a subject, for at least one pair of contralateral limb segments, and, in the context of gait analysis, preferably for both the thighs and shanks of the subject. For example, the motion data may comprise four signals, including a first signal representing continual (e.g., 60 Hz) measurements of sagittal angular velocity by a first IMU 212 positioned on the subject's right thigh, a second signal representing continual measurements of sagittal angular velocity by a second IMU 212 positioned on the subject's right shank, a third signal representing continual measurements of sagittal angular velocity by a third IMU 212 positioned on the subject's left thigh, and a fourth signal representing continual measurements of sagittal angular velocity by a fourth IMU 212 positioned on the subject's left shank. In the context of arm-motion analysis, the signals may include a first signal representing continual (e.g., 60 Hz) measurements of sagittal angular velocity by a first IMU 212 positioned on the subject's right upper arm, a second signal representing continual measurements of sagittal angular velocity by a second IMU 212 positioned on the subject's right forearm, a third signal representing continual measurements of sagittal angular velocity by a third IMU 212 positioned on the subject's left upper arm, and a fourth signal representing continual measurements of sagittal angular velocity by a fourth IMU 212 positioned on the subject's left forearm.

In subprocess 420, the motion data received in subprocess 410 are segmented into individual motion segments. In the context of gait analysis, these motion segments may represent strides, and therefore, will be primarily referred to herein as "strides." However, it should be understood that, even though the term "strides" is used, the motions segments may represent distinct repetitions of any movement, such as a particular arm movement (e.g., forward swing, backward swing, or forward and backward swing of the arm, a reach out and pull back of the arm, one rotation of the wrist, forearm, or shoulder, etc.). For simplicity, the term "stride" will be used to refer to any repeating motion segment, whether it is a stride of the legs or other leg movement, or an arm movement.

In an embodiment, each signal, representing sagittal angular velocity from one IMU 212, is divided into its constituent stride signals. Using the example above, each of the first, second, third, and fourth signals may be segmented into a plurality of stride signals. It should be understood that each of the plurality of stride signals is a segment of the signal that represents a single stride or other motion segment. In the context of gait analysis, a stride may be defined as the segment of signal from a toe-off (i.e., a point at which the toe of the limb leaves the ground) to a next toe-off of the respective limb. However, the strides may be defined in an alternative manner, as long as the definition of the stride is consistent across all stride signals to be aligned and compared.

The segmentation of signals into stride signals in subprocess 420 may be performed in any manner. For example, in a signal of sagittal angular velocity of a lower limb segment, each swing phase is represented by a positive peak with local maxima and is followed by a stance phase as the subject rolls the foot from heel to toe. Negative peaks in the signal represent the change in foot rotation during the stance phase. Thus, the signals may be segmented into a plurality of stride signals by identifying each negative peak (i.e., representing the end of a stride) before a swing phase (i.e., representing the start of a new stride). In other words, each stride signal starts at a negative peak in the signal and ends at the next negative peak in the signal. In this manner, a plurality of consecutive stride signal segments may be identified in each signal of sagittal angular velocity.

In subprocess 430, each pair of contralateral strides may be aligned. Pairs of contralateral stride signals may be identified by pairing a first stride signal, segmented from a signal for a limb segment (e.g., thigh or shank) on one side of the subject (e.g., left or right), with a second contralateral stride signal, segmented from a signal for the corresponding limb segment (e.g., thigh or shank) on the opposite side of the subject (e.g., right or left), that is adjacent in time (i.e., occurring immediately before, after, or overlapping the first stride in the time dimension of the signal). It should be understood that in an embodiment which acquires signals from both the thigh and shank, thigh movements will be represented by pairs of contralateral stride signals acquired from the thighs, and shank movement will be represented by pairs of contralateral stride signals acquired from the shanks. Similarly, in an embodiment which acquires signals from both the upper arm and forearm, upper arm movements will be represented by pairs of contralateral stride signals acquired from the upper arms, and forearm movements will be represented by pairs of contralateral stride signals acquired from the forearms.

As mentioned elsewhere herein, the alignment in subprocess 430 may comprise DTW. For example, DTW may be used to stretch the two stride signals representing the pair of contralateral strides onto a common set of points, such that the sum of the Euclidean distances between corresponding points is minimized. DTW is a technique for comparing time series data when the time indices between data points to be compared are not synchronized. One example implementation of a DTW program package is available from the Journal of Statistical Software (www.jstatsoft.org/article/view/-v031i07). Another example implementation of DTW is the "dtw" function provided in MATLAB™, which is a product of The MathWorks, Inc. of Natick, Massachusetts (www.mathworks.com/help/signal/ref/dtw.html).

In subprocess 440, a stride difference may be calculated for the pairs of aligned contralateral stride signals output by subprocess 430. For example, for each pair of aligned contralateral stride signals, the sum of Euclidean distances between each aligned pair of points in the two stride signals may be calculated. In an embodiment, this sum of Euclidean distances between two stride signals $X$ and $Y$ may be calculated using the root sum of squared differences $d_{mn}(X, Y)$, which represents the difference between the $m$-th sample ($x_m$) of stride signal $X$ and the n-th sample ($y_n$) of stride signal $Y$ according to the following equation:

$$d_{mn}(X,Y) = \sqrt{(x_m - y_n) * (x_m - y_n)}$$

This sum of Euclidean distances may be used as or to derive a motion difference symmetry (MDS) for each pair of aligned contralateral stride signals. MDS for a pair of contralateral strides represents the similarities or differences between the angular motion (e.g., the sagittal angular velocities) for that pair of contralateral strides.

MDS may be measured in degrees per second (deg/s) and represent a quantitative measure of symmetry between contralateral strides, with lower values of MDS representing better symmetry (e.g., more similarities and/or fewer differences in angular velocities between contralateral strides) and higher values of MDS representing less symmetry (e.g., fewer similarities and/or more differences in angular velocities between contralateral strides). An MDS value of zero represents the highest possible level of symmetry. It should be understood that MDS can be calculated independently for contralateral strides of both the thigh and the shank and/or the upper arm and the forearm.

In subprocess 450, a symmetry metric (e.g., gait symmetry metric) is output. In an embodiment, the symmetry metric that is output in subprocess 450 is the mean MDS and/or SSS. The mean MDS is the average value of the MDS across all pairs of contralateral stride signals in the motion data. Thus, the mean MDS represents the overall symmetry (e.g., gait symmetry) for a subject across an entire motion test (e.g., ambulation test). It should be understood that mean MDS may be calculated independently for the thigh and the shank and/or the upper arm and the forearm, or may be combined (e.g., averaged) into a single MDS value that represents an overall symmetry for both the thigh and the shank and/or the upper arm and the forearm.

In an embodiment, SSS is derived from the MDS as follows:

$$SSS = 100 - \left(100 \times \frac{mean\ MDS}{threshold_s}\right)$$

wherein $threshold_s$ is an MDS value that represents an extreme (e.g., maximum) level of asymmetry. This threshold may be chosen empirically. For example, in experiments of gait analysis, the most extreme level of asymmetry was exhibited by a subject with a transfemoral amputation, who had an MDS of 254.1. Since it is unknown what the maximum value of MDS is among the entire population of amputees, $threshold_s$ may be chosen by consensus. In an embodiment, $threshold_s$

equals 300, which is well above the mean MDS exhibited for both thighs and shanks in experiments and surpasses the maximum observed MDS. However, any appropriate value of $threshold_s$ may be used. It should be understood that the value of $threshold_s$ for arm motion analysis may be chosen in a similar manner and will generally be different than the value of $threshold_s$ for gait analysis.

Notably, an SSS of 100% represents the highest possible symmetry between contralateral limb segments. SSS can be thought of as a clinically friendly score of symmetry, since it converts the MDS values to a scale (i.e., from approximately zero to one hundred) that is easier to understand and able to be immediately grasped. It should be understood that SSS may be calculated independently for the thigh and the shank and/or the upper arm and the forearm, or may be combined (e.g., averaged) into a single SSS value that represents an overall symmetry for both the thigh and the shank and/or the upper arm and the forearm.

2.2.2. Repeatability (SRS)

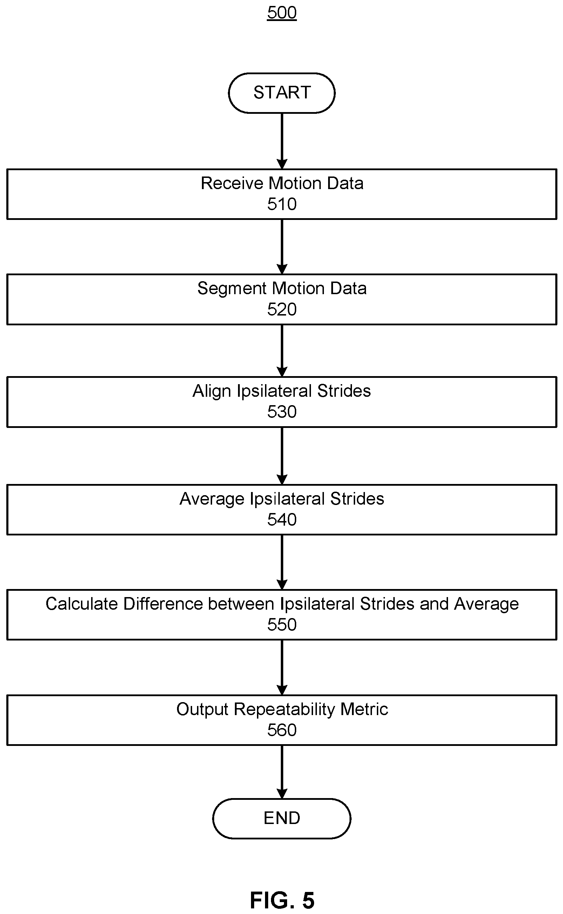

FIG. 5 illustrates a process 500 for calculating a repeatability metric (e.g., gait repeatability metric), such as SRS, according to an embodiment. Process 500 may be implemented by the disclosed application, which may be executed by an external processing system 100 that is remote from sensor system 200 and receives motion data from sensor system 200 via wireless or wired communication. Alternatively, the application may be executed by an onboard processing system 100 that is local to sensor system 200. In an embodiment which implements both processes 400 and 500 to compute both a symmetric metric and a repeatability metric, processes 400 and 500 may be computed by the same application on the same processing system 100, whether external or local to sensor system 200.

Subprocesses 510 and/or 520 in process 500 may be similar or identical to subprocesses 410 and/or 420 in process 400. Thus, a description of these subprocesses will be omitted as redundant. In an embodiment which calculates both SSS and SRS, subprocesses 410 and 510 may be implemented as a single subprocess that provides a single output, and subprocesses 420 and 520 may be implemented as a single subprocess that receives that output and then provides a single output to both of subprocesses 430 and 530.

In subprocess 530, the ipsilateral stride signals for each limb segment may be aligned. The alignment in subprocess 530 may be similar or identical to the alignment in subprocess 430. For example, the alignment of subprocess 530 may utilize DTW to align the signal segments representing the set of ipsilateral strides for a given limb segment.

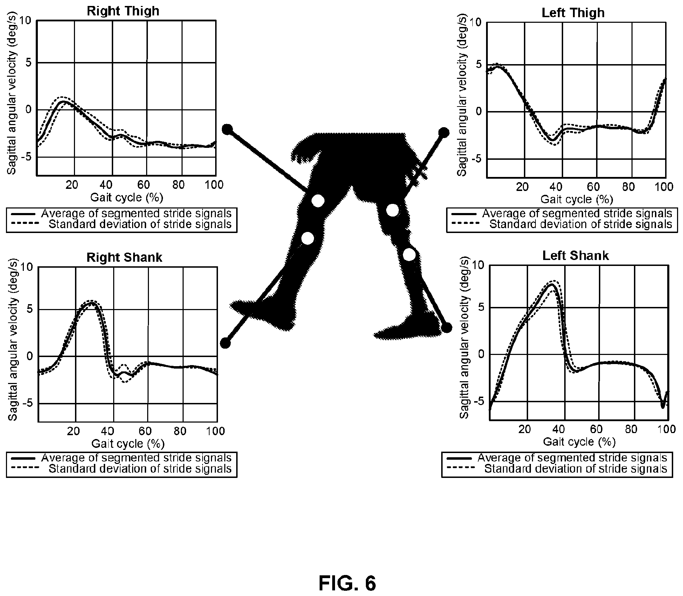

In subprocess 540, for each limb segment (e.g., right thigh, right shank, left thigh, left shank, right upper arm, right forearm, left upper arm, or left forearm), the ipsilateral stride signals, as aligned in subprocess 530, may be averaged across all of the strides for that limb segment. This results in an average stride signal for each limb segment. FIG. 6 illustrates an example of the average stride signals for each of the right thigh, right shank, left thigh, and left shank, acquired by sensor system 200, during an ambulation test of a subject with a transfemoral amputation, according to an embodiment. The average stride signal across all segmented ipsilateral strides is illustrated by the solid line in each signal graph, whereas the standard deviation is illustrated by the dashed lines in each signal graph.

In subprocess 550, the stride difference between each consecutive stride signal for each limb segment and the average stride signal for the limb segment may be calculated. Each stride difference may be calculated in a similar or identical manner as the stride differences calculated in subprocess 440. For example, the sum of Euclidean distances between the points in each ipsilateral stride signal, within the signal for a limb segment, and the respective aligned points in the average stride signal for that limb segment may be calculated.

This sum of Euclidean distances may be used as or to derive a motion difference repeatability (MDR) for each ipsilateral stride signal. MDR for a stride represents the similarities or differences between the sagittal angular velocities of that stride and the other ipsilateral strides.

MDR may be calculated in a similar or identical manner as MDS. In an embodiment, the only difference is that MDS is calculated between contralateral stride signals, whereas MDR is calculated between a stride signal and its ipsilateral average stride signal. Accordingly, MDR may also be measured in degrees per second, but represents a quantitative measure of repeatability, with lower values of MDR representing better repeatability (e.g., more similarities and/or fewer differences in angular velocities between ipsilateral strides) and higher values of MDR representing less repeatability (e.g., fewer similarities and/or more differences in angular velocities between ipsilateral strides). An MDR value of zero represents the highest possible level of repeatability. It should be understood that MDR may be calculated independently for each limb segment.

In subprocess 560, a repeatability metric (e.g., gait repeatability metric) is output. In an embodiment, the repeatability metric that is output in subprocess 560 is the mean MDR and/or SRS. The mean MDR is the average value of the MDR across all ipsilateral strides in the motion data for a given limb segment. Thus, the mean MDR represents the overall repeatability for a given limb segment of a subject. It should be understood that mean MDR may be calculated independently for each limb segment (e.g., right thigh, right shank, left thigh, left shank, right upper arm, right forearm, left upper arm, or left forearm), or may be combined (e.g., averaged) into a single MDR value that represents an overall repeatability for all limb segments.

In an embodiment, SRS is derived from the MDR as follows:

$$SRS = 100 - \left(100 \times \frac{mean\ MDR}{threshold_R}\right)$$

wherein $threshold_R$ is an MDR value that represents an extreme (e.g., maximum) level of unrepeatability. As with $threshold_S$, this threshold may be chosen empirically. Since it is unknown what the maximum value of MDR is among the entire population of amputees, $threshold_R$ may be chosen by consensus. In an embodiment of gait analysis, $threshold_R$ equals 30, which is well above the average MDR exhibited for thighs and shanks in experiments and surpasses the maximum observed MDR. However, any appropriate value of $threshold_R$ may be used. It should be understood that the value of $threshold_R$ for arm motion analysis may be chosen in a similar manner and will generally be different than the value of $threshold_R$ for gait analysis.

Notably, an SRS of 100% represents the highest possible repeatability for a limb segment. SRS can be thought of as a clinically friendly score of repeatability, since it converts the MDR values to a scale (i.e., from approximately zero to one hundred) that is easier to understand and able to be immediately grasped. It should be understood that SRS may be calculated independently for each limb segment, or may be combined (e.g., averaged) into a single SRS value that represents an overall repeatability for all limb segments.

In some cases, the MDR and/or SRS may be specified according to whether or not the metric is for a prosthetic or intact limb segment. For example, for a unilateral transfemoral amputee, a separate MDR and/or SRS may be provided for the prosthetic thigh, prosthetic shank, intact thigh, and intact shank. For a unilateral transtibial amputee, a separate MDR and/or SRS may be provided for the left thigh, right thigh, prosthetic shank, and intact shank.

## 3. Experiments

In a particular experiment, the disclosed application was used to investigate segmental symmetry and repeatability in the gaits of subjects with unilateral lower limb amputations. Each of the subjects had experienced a unilateral transtibial (TT) or transfemoral (TF) amputation, was between 18-80 years of age, had been using a prosthesis for at least three months, could ambulate independently, reported a comfortable socket fit with the prosthesis, and did not have open wounds or multiple amputations. One-hundred-twenty-eight subjects (n=128) were enrolled, with a mean age of 49.5 years and a standard deviation of 15.2 years, and a mean time since amputation of 9.8 years (with a standard deviation of 10.8 years). The demographics and medical histories of these subjects were recorded, and the performances of these subjects' intact and prosthetic lower limbs were examined. Descriptive characteristics of the subjects of the experiment are detailed in the table below:

| Characteristic | TT Amputees (n=65) | TF Amputees (n=63) |
|---|---|---|
| Gender | | |
|     Male | 34 (26.6%) | 27 (21.1%) |
|     Female | 31 (14.2%) | 36 (28.1%) |
| Side of Amputation | | |
|     Right | 39 (30.5%) | 27 (21.1%) |
|     Left | 26 (20.3%) | 36 (28.1%) |
| Mean Age | 51.0 years (std dev. = 14.1) | 47.9 years (std. dev. = 16.2) |
| Mean Time Since Amputation | 8.5 years (std. dev. = 8.4) | 11.1 years (std. dev. = 12.8) |

## 3.1. Data Collection

When necessary, subjects were assisted with donning sensor system 200, comprising elastic knee sleeves 210A and 210B with a total of four IMUs 212 that were validated for measuring angular velocity during gait, as described in "Characterizing the impact of sampling rate and filter design on the morphology of lower limb angular velocities," by Allseits et al., IEEE Sensors Journal, 2019, and "The development and concurrent validity of a real-time algorithm for temporal gait analysis using inertial measurement units," by Allseits et al., J. Biomech., 2017, 55:27-33. Positioning of the inertial sensors of IMUs 212, in proximity to the subjects' knee joints, was calibrated using an alignment trial, as detailed in "A practical step length algorithm using lower limb angular velocities," by Allseits et al., J. Biomech., 2018, 66:137-44. In the alignment trial, a subject walked ten steps in a straight line. Knee sleeves 210 enabled placement of the inertial sensors at the same relative body locations across all subjects and the ability to obtain accurate segmental angular velocities in the sagittal plane.

Once calibration was complete, each subject performed three trials of a ten-meter walk test at a self-selected walking speed on a Zeno™ Electronic Walkway System (Model Z4 x 14) by ProtoKinetics LLC of Havertown, Pennsylvania, United States. Subjects walked a measured distance of ten meters, with the initial two meters and the final two meters used as acceleration and deceleration zones, respectively. Thus, for each subject, raw motion data was collected for the middle six meters of each trial to produce the gait data for that subject, and the gait metrics were averaged across all three trials.

As the subjects walked, wireless IMUs 212 collected the raw motion data, comprising angular velocities along three perpendicular axes, at a sampling frequency of 60 Hz, and transmitted the raw motion data to a tablet computer (e.g., iPad™) via Bluetooth Low Energy. The raw motion data was then wirelessly transmitted from the tablet computer, through a Wi-Fi™ network, to a server system where it was stored in a secure PostgresSQL database.

3.2. Gait Analysis

SSS and SRS were computed, according to processes 400 and 500, respectively, for each subject using MATLAB™ R2017b. Across multiple trials of the ten-meter walk test, SSS demonstrated excellent test-retest reliability and narrow confidence intervals, and SRS exhibited good to excellent reliability. The experiment demonstrated that SSS and SRS are stable and repeatable functional outcome metrics.

Specifically, intraclass correlation coefficients (ICCs) were calculated to determine the test-retest reliability of SSS and SRS across the multiple trials of the ten-meter walk test, as detailed in the table below:

| Variable | ICC (95% Confidence Interval) | F statistic | p value |
|---|---|---|---|
| Thigh SSS | 0.97 (0.95-0.98) | 0.28 | 0.75 |
| Shank SSS | 0.99 (0.98-0.99) | 0.50 | 0.61 |
| Thigh SRS Prosthetic | 0.71 (0.61-0.79) | 0.87 | 0.42 |
| Thigh SRS Intact | 0.73 (0.64-0.80) | 2.30 | 0.10 |
| Shank SRS Prosthetic | 0.85 (0.80-0.89) | 0.32 | 0.73 |
| Shank SRS Intact | 0.72 (0.62-0.80) | 0.21 | 0.81 |

The intraclass correlation coefficients were calculated using a two-way mixed effects model with absolute agreement derived from repeated measures analysis of variance for each symmetry and repeatability variable. Utilization of intraclass correlation coefficients, with nomenclature (2, k) and 95% confidence intervals, were used to determine the test-retest reliability, based on the description in "Intraclass correlations: uses in assessing rater reliability," by Shrout et al., Pscyhol. Bull., 1979, 86(2):420-8. As a general guideline, intraclass correlation coefficients below 0.50 indicate poor reliability, intraclass correlation coefficients between 0.50-0.75 indicate moderate reliability, and intraclass correlation coefficients above 0.75 indicate good reliability. Acceptable reliability depends on the intended purpose for the metric. However, to ensure practical validity for clinical metrics, intraclass correlation coefficients greater than 0.90 are preferred.

The normality of data was determined by visual examination of Quantile-Quantile plots and the results of Shapiro-Wilk testing, as described in "Power comparisons of Shapiro-Wilk, Kolmogorov-Smirnov, Lilliefors and Anderson-Darling test," by Razali et al., J. Stat. Model Analytics, 2011, 2(1):21-33. Descriptive statistics were calculated for the sample population. One-tailed non-parametric statistics were used to analyze differences between groups based on amputation level, as well as within groups, for symmetry and repeatability variables of bilateral thighs and shanks. Post-hoc Bonferonni adjustments were utilized to protect against type I error, and adjusted p values were used to determine statistical significance.

3.3. Results

The gait symmetry (represented by the SSS) and repeatability (represented by the SRS) for the subjects were used to detect biomechanical differences between the prosthetic and intact lower limb segments of the subjects. For example, differences in walking patterns between transfemoral amputees and transtibial amputees were examined. Notably, greater asymmetries and more inconsistent movements were exhibited by subjects with transfemoral amputations than by subjects with transtibial amputation ($p < 0.004$, Cohen's d = 0.65-1.1). The various metrics obtained for the subjects are detailed in the table below:

| Variable | TT Amputees mean (std. dev.) [range] | TF Amputees mean (std. dev.) [range] | p value |
|---|---|---|---|
| Age (years) | 51.1 (14.1) [21.7-77.2] | 47.9 (16.2) [19.1-77.4] | 0.2 |
| Time Since Amputation (years) | 8.5 (8.4) [0.78-50.0] | 11.1 (12.8) [0.3-49.4] | 0.3 |
| Thigh MDS (deg/s) | 13.8 (13.8) [1.7-66.9] | 46.7 (39.7) [7.8-254.1] | <0.0001* |
| Thigh SSS (%) | 93.5 (6.0) [72.9-99.7] | 86.5 (14.0) [15.3-99.3] | <0.0001* |
| Shank MDS (deg/s) | 16.8 (14.3) [2.1-78.4] | 32.4 (26.3) [3.8-152.1] | <0.0001* |
| Shank SSS (%) | 93.5 (7.0) [58.5-99.7] | 90.5 (7.7) [49.3-98.0] | 0.0002* |
| Thigh MDR Prosthetic (deg/s) | 0.88 (0.7) [0.24-3.2] | 2.0 (1.8) [0.4-13.4] | <0.0001* |
| Thigh SRS Prosthetic (%) | 96.2 (3.4) [84.2-99.2] | 94.2 (6.1) [55.2-99.0] | 0.002* |
| Thigh MDR Intact (deg/s) | 0.87 (0.5) [0.23-2.9] | 1.5 (1.1) [0.3-6.0] | <0.0001* |
| Thigh SRS Intact (%) | 96.5 (2.8) [83.1-99.2] | 95.9 (3.1) [80.1-98.6] | 0.01 |
| Shank MDR Prosthetic (deg/s) | 2.1 (1.0) [1.0-5.2] | 2.4 (3.0) [0.72-24.6] | 0.5 |
| Shank SRS Prosthetic (%) | 93.4 (2.7) [84.8-97.3] | 91.7 (10.2) [18.2-97.6] | 0.4 |
| Shank MDR Intact (deg/s) | 1.6 (0.5) [0.9-3.4] | 2.2 (1.2) [1.0-7.7] | <0.0001* |
| Shank SRS Intact (%) | 94.7 (1.7) [88.7-97.1] | 92.6 (4.1) [74.4-96.7] | <0.0001* |
| *Indicates significant difference after Bonferonni adjustment at p $\leq$ 0.004 | | | |

SSS and SRS measures were also computed for thirteen able-bodied adult subjects, resulting in a mean SSS of 99.1% and a mean SRS of 97.9%. These findings indicate apparent differences in movement patterns between amputees and able-bodied subjects.

The results revealed that subjects with unilateral lower limb amputations walk with asymmetrical and inconsistent movements of their lower limbs, as demonstrated by lower SSS and SRS values than the able-bodied subjects. The results also revealed that differences exist in SSS and SRS values between subjects with transtibial amputations and subjects with transfemoral amputations. Thus, the use of SSS to measure gait symmetry and/or SRS to measure gait repeatability provides clinically useful means to reliably capture movement asymmetries of the lower limbs that were not previously apparent using typical temporal-spatial gait parameters and were not previously quantifiable with clinician observation. Specifically, the results showed that SSS and SRS are able to discern and quantify the extent of gait symmetry and repeatability of the thigh and shank motions, during ambulation tests, between subjects with different levels of amputation.

### 3.3.1. Across Groups

Transtibial and transfemoral amputees exhibited differences in gait symmetry and repeatability for both the thigh and the shank. Specifically, differences were found in symmetry for both the thigh (i.e., mean thigh MDS of 13.8 degrees/second (deg/s) vs. 46.7 deg/s, with Cohen's d=1.11) and the shank (i.e., mean shank MDS of 16.8 deg/s vs. 32.4 deg/s, with Cohen's d=0.74), resulting in a thigh SSS of 93.5% for transtibial amputees compared to a thigh SSS of 86.5% for transfemoral amputees, and a shank SSS of 93.5% for transtibial amputees compared to a shank SSS of 90.5% for transfemoral amputees. Differences were also found in repeatability for both the thigh and shank, as demonstrated by the MDR and SRS metrics. Transtibial and transfemoral amputees exhibited differences in all variables and corresponding moderate to large effect sizes (Cohen's d = 0.65-0.82), with the exception of SRS for the intact thigh segment (96.5% vs. 95.9%) and the MDR and SRS for the prosthetic shank (2.1 deg/s vs. 2.4 deg/s and 93.4% vs. 91.7%, respectively).

In contrast, the use of an instrumented walkway to compute traditional temporal-spatial gait parameters demonstrated only one significant difference between transtibial and transfemoral amputees. Specifically, traditional gait parameters only reveal that transfemoral amputees spend more time in stance on the intact limb than transtibial amputees, as demonstrated in the table below:

| Variable | TT Amputees (n=30) mean (std. dev.) [range] | TF Amputees (n=30) mean (std. dev.) [range] | p value |
|---|---|---|---|
| Prosthetic Stance Time (seconds) | 0.7 (0.08) [0.5-0.96] | 0.73 (0.12) [0.60-1.16] | 0.12 |

(continued)

| Variable | TT Amputees (n=30) mean (std. dev.) [range] | TF Amputees (n=30) mean (std. dev.) [range] | p value |
|---|---|---|---|
| Intact Stance Time (seconds) | 0.73 (0.08) [0.56-0.96] | 0.83 (0.17) [0.63-1.34] | 0.005** |
| Prosthetic Step Length* | 0.42 (0.04) [0.32-0.49] | 0.39 (0.07) [0.22-0.48] | 0.12 |
| Intact Step Length* | 0.4 (0.04) [0.29-0.47] | 0.39 (0.05) [0.25-0.46] | 0.47 |
| Prosthetic Initial Double Limb Stance (%) | 15.5 (2.21) [11.6-20.5] | 14.78 (2.95) [10.6-24.4] | 0.10 |
| Intact Initial Double Limb Stance (%) | 13.13 (1.95) [7.9-17.82] | 13.68 (2.66) [9.55-20.28] | 0.90 |
| Prosthetic Terminal Double Limb Stance (%) | 13.26 (1.90) [7.82-17.98] | 13.85 (2.69) [9.58-20.40] | 0.97 |
| Intact Terminal Double Limb Stance (%) | 15.76 (2.15) [11.62-20.53] | 14.96 (2.84) [10.89-23.80] | 0.08 |
| *In centimeters divided by the subject's height to normalize the value<br>**Indicates significant difference after Bonferonni adjustment at $p \leq 0.006$ (0.05/8) p values are two-tailed hypotheses | | | |

Temporal-spatial gait parameters can be derived from IMUs placed on bilateral thighs and shanks using the double-pendulum model described in "Gait assessment in Parkinson's disease: toward an ambulatory system for long-term monitoring," by Salarian et al., IEEE Trans. Biomed. Eng., 2004, 51(8):1434-43, "Detection of spatio-temporal gait parameters by using wearable motion sensors," by Lee et al., Conf. Proc. IEEE Eng. Med. Biol. Soc., 2005, 7:6836-9, and Allseits et al. 2017. However, to calculate metrics such as stride length and stance time, the motions of the thigh and shank may be combined, in order to negate the independent contribution of each lower limb segment.

Notably, IMUs have not previously been used to compare motions of bilateral thighs and shanks during walking. The disclosed gait analysis revealed that thigh and shank movements exhibited differences based on the amputation level of a subject. Specifically, subjects with unilateral transfemoral amputations demonstrated larger differences in angular velocities of the prosthetic thigh and shank compared to the intact thigh and shank during a linear gait. This resulted in significantly lower thigh and shank SSS's in comparison to subjects with unilateral transtibial amputations.

Coordinated motion of bilateral thighs and shanks is integral to a normal gait pattern, as confirmed by "Motor control: translating research into clinical practice," by Shumway-Cook et al., 5th ed., Lippincott Williams & Wilkens, 2017. However, due to the nature of the amputation at the transfemoral level, the distal muscle attachments of the thigh are interrupted, and a prosthetic knee joint replaces the anatomical knee joint that was previously controlled by the long muscles of the thigh. The combination of reduced limb length and hip muscle atrophy of the amputated limb results in strength and motor control imbalances, as described in "Prosthetic gait of unilateral transfemoral amputees: a kinematic study," by Jaegers et al., Arch. Phys. Med. Rehabil., 1995, 76(8):736-43.

Moreover, stability during walking may be affected by altered movement, resulting from the limb-socket interaction, which can depend on the residual limb anatomy, as discussed in "Pistoning assessment in lower limb prosthetic sockets," by Eshraghi et al., Prosthet. Orthot. Int., 2012, 36(1):15-24, and the type of suspension used to hold the socket to the thigh, as discussed in "The evidence-base for elevated vacuum in lower limb prosthetics: literature review and professional feedback," by Gholizadeh et al., Clin. Biomech., 2016, 37:108-16. Therefore, performance of the prosthetic limb in transfemoral amputees during walking can be partially controlled by the characteristics of the prosthetic knee and foot components, as well as the socket design and suspension system, which may all influence movement of the lower limb segments.

A multitude of socket designs currently exist within the practice of prosthetics. However, no particular design has garnered a unanimous recommendation. In addition, various stance and swing control mechanisms exist in current prosthetic knee technology. Each has different internal cadence mediums and adjustment controls for the knee during swing and stance to accommodate the amputee's gait. These differences may explain the resulting range of values and large standard deviation in the SRS for the prosthetic limb shank of subjects with transfemoral amputations, which, in turn, may have obscured statistical differences in this value between transtibial and transfemoral amputees. The decisions for adapting socket design and prosthetic knee control to a specific subject's gait relies on the discretions of the subject's prosthetist, and little standardization exists with regard to which prosthetic design is optimal in reducing gait asymmetry, gait repeatability, or other gait deviations.

As mentioned above, traditional temporal-spatial gait parameters, including percentages of the gait cycle, recorded on an electronic walkway during a ten-meter walk test, are unable to detect gait differences between transtibial and transfemoral amputees. In addition, despite providing objective data with selected gait parameters, gait mats can only calculate gait variables through plantar pressure measurements, resulting from footfalls on the surface of the gait mat. Additional instrumentation would be required to measure the movement of lower limb segments. Thus, a gait mat would be unable to detect compensatory or abnormal movements to progress the lower limbs for subjects that exhibit symmetrical step lengths.

Observational Gait Analysis (OGA) may be performed by clinicians, as it is economical and efficient in the clinical setting. Although OGA is comprehensive in its approach of head-to-toe evaluation of a subject's gait, it is a subjective metric that is subject to greater variability and dependent on the observing clinician's experience and background.

Advantageously, SSS and SRS can augment commonly performed OGA and/or other gait measurement techniques by providing objective, quantitative movement data to represent a clinician's observations. Additionally, measurement of bilateral thigh and shank symmetry using SSS was able to discriminate between amputation levels in community-ambulating subjects with lower limb amputations. This finding, along with excellent test-retest reliability, suggests that subjects' gaits can be objectively and quantitatively described using segmental analysis to determine the differences between limbs. Thus, the disclosed application provides a clinically friendly tool for comprehensive pathological gait analysis.

### 3.3.2. **Within** Groups

Comparisons were also performed between subjects with the same level of amputation (i.e., within the transtibial cohort and within the transfemoral cohort). Significant differences in repeatability between intact and prosthetic limbs were exhibited within both the transtibial and transfemoral cohorts, as illustrated in the table below:

| Subject | Variable | Prosthetic Limb mean (std. dev.) [range] | Intact Limb mean (std. dev.) [range] | p value |
|---|---|---|---|---|
| TT Amputees | Thigh MDR | 0.88 (0.7) [0.24-3.2] | 0.87 (0.5) [0.23-2.9] | 0.3 |
| | Thigh SRS | 96.2 (3.4) [84.2-99.2] | 96.5 (2.8) [83.1-99.2] | 0.8 |
| | Shank MDR | 2.1 (1.0) [1.0-5.2] | 1.6 (0.5) [0.9-3.4] | <0.0001* |
| | Shank SRS | 93.4 (2.7) [84.8-97.3] | 94.7 (1.7) [88.7-97.1] | 0.0004* |
| TF Amputees | Thigh MDR | 2.0 (1.8) [0.4-13.4] | 1.5 (1.1) [0.3-6.0] | 0.02* |
| | Thigh SRS | 94.2 (6.1) [55.2-99.0] | 95.9 (3.1) [80.1-98.6] | 0.2 |
| | Shank MDR | 2.4 (3.0) [0.72-24.6] | 2.2 (1.2) [1.0-7.7] | 0.3 |
| | Shank SRS | 91.7 (10.2) [18.2-97.6] | 92.6 (4.1) [74.4-96.7] | 0.5 |
| *Indicates significant difference after Bonferonni adjustment at $p \leq 0.05$ | | | | |

Within the group of transtibial amputees, there was a significant difference between the intact and prosthetic mean shank MDR (i.e., 1.6 deg/s vs. 2.1 deg/s) and mean SRS (i.e., 94.7% vs. 93.4%). However, there was no similar difference between the intact and prosthetic mean thigh MDR or SRS. Transversely, within the group of transfemoral amputees, there was a significant difference between the intact and prosthetic mean thigh MDR (i.e., 1.5 deg/s vs. 2.0 deg/s). However, there was no similar difference between the intact and prosthetic mean thigh SRS, and no statistical difference between the intact and prosthetic mean shank MDR or SRS.

Thus, the transtibial cohort presented greater inconsistency of movement between the prosthetic shank and intact shank, as demonstrated by higher shank MDR and lower shank SRS for the prosthetic shank. This could reflect deficient motor control due to lack of anatomy, non-optimal socket fit, and/or properties of the prosthetic foot.

The transfemoral cohort presented with greater inconsistency of movement between each prosthetic and intact segment, as demonstrated by higher MDR and lower SRS for both prosthetic segments. Statistical analysis did not indicate that there were significant differences between prosthetic and intact limb performance in transfemoral amputees. However, the variability in the SRS range of both prosthetic segments, compared to the intact segments, suggests the existence of underlying factors that may influence the movement pattern as transfemoral amputees control their prosthetic limb during forward progression.

Changes in motor control or coordination of the lower limb, joint motion limitations, and/or possible effects of prosthetic socket design may contribute to segmental gait repeatability. In amputation surgery, there is inherent loss of muscle, bone, and joint tissues. This often alters strength and balance during ambulation with a prosthesis, as documented in Jaegers et al. and "Mechanical work adaptations of above-knee amputee ambulation," by Seroussi et al., Arch. Phys. Med. Rehabil., 1996, 77(11):1209-14. The inability to restore muscle control to similar pre-amputation capabilities may result in inconsistent residual limb movement patterns during walking. These irregularities in movement could be associated with limb length, time since amputation, age, strength, range of motion, debility, and/or prosthetic training.

SRS may help identify the underlying impairments that reduce the repeatability of movement within the lower limb of amputees, and guide specific therapeutic interventions to promote repeatability and symmetry of movement for a more efficient and safer gait. The IMU-based metrics of SSS and SRS have strong clinical implications as a means of objective gait analysis, classification of gait deviations, and/or identification of underlying impairments.

4. <u>Example Use Cases</u>

As disclosed herein, the sensor-based measurement of segmental gait symmetry and repeatability is reliable in detecting differences in movement between the prosthetic lower limb and the intact lower limb of a lower limb amputee. Thus, the disclosed processes represent a convenient tool for enhanced prosthetic gait analysis, and have the potential to focus rehabilitative and prosthetic interventions. The benefits of such gait analysis include, without limitation, determining when pre-injury function has been restored to a lower limb (e.g., orthopedic, neurological, and age-related injuries), determining if rehabilitation (e.g., gait training) is required and/or how long rehabilitation is required, preventing or reducing falls, reducing secondary health conditions resulting from gait asymmetry over time, assessing prosthetic gait, assessing orthotic gait, assessing and/or treating numerous other gait-related conditions, and/or the like.

The assessment of subjects with unilateral lower limb amputations, using the disclosed gait symmetry and/or repeatability metrics, provides clinically applicable information related to lower limb movement patterns that can augment or replace traditional gait metrics and discriminate between subjects with different levels of amputation. In addition, the disclosed SSS and SRS metrics are clinically friendly measures of gait performance that quantify lower limb movements with confidence and exhibit excellent test-retest reliability.

Beyond the specific example of lower limb amputees, the disclosed systems and processes may be used in many contexts and for many types of subjects. For example, the disclosed systems and processes may be used by hospitals, health clinics, rehabilitation centers, athletic teams, prosthetic clinics, nursing homes, physical screenings, and in any other context in which gait analysis is appropriate. Subjects who may benefit from such gait analysis include, without limitation, patients with lower limb or spinal injuries or degenerative diseases, patients undergoing physical therapy, patients recovering from joint surgery, athletes, the elderly, and/or the like. The disclosed gait symmetry and/or repeatability metrics (e.g., MDS, SSS, MDR, and/or SRS) may be provided to such subjects as feedback on their gait performances.

As mentioned throughout, the symmetry and repeatability metrics may also be used to objectively quantify symmetry and repeatability in upper limbs, such as the arms. In this case, SSS represents the similarity of angular motion (e.g., sagittal angular velocity) of the arm and/or forearm between contralateral motion segments (e.g., swings). Similarly, in this case, SRS represents the similarity of angular motion (e.g., sagittal angular velocity) of the arm and/or forearm across consecutive ipsilateral motion segments (e.g., swings).

In a clinical setting, SSS and SRS may be implemented as a simple-to-use mobile wireless sensor system for objectively quantifying the symmetry and/or repeatability of human movement and assessing balance for a wide variety of patient populations. Use of this system can reduce rehabilitation time, increase efficiency, assist with clinical decision-making, improve clinical documentation, and/or reduce overall clinical costs. Examples of patient populations which may benefit from the disclosed system and application include, without limitation, those with:

- Neurological disorders, such as lower motor neuron (LMN) and/or upper motor neuron (UMN) diseases (e.g., spinal cord injury, multiple sclerosis, stroke, traumatic brain injury (TBI), amyotrophic lateral sclerosis (ALS), etc.), systemic and progressive diseases (e.g., Guillain-Barre syndrome, muscular dystrophy, etc.), and balance impairment (e.g., geriatric, vestibular, proprioceptive impairment, UMN-related conditions, etc.).
- Musculoskeletal disorders, such as degenerative joint disease (e.g., osteoarthritis, rheumatoid arthritis, etc.), post-surgical recovery (e.g., total knee arthroplasty (TKA), total hip arthroplasty (THA), spinal surgery, hip fracture, etc.), spinal pain (e.g., disc, acute trauma, chronic pain, etc.), peripheral nerve injury (e.g., nerve lesion recovery or permanent), and muscle injury (e.g., contusion, laceration, tears, avulsions, etc.).
- Clinical sport and/or performance enhancement, such as rehabilitation progression, return to activity, and return to sport.

Both SSS and SRS testing can be administered concurrently with established measures of mobility, such as:

- Ten-meter walk test: SSS and/or SRS of the lower limbs can be computed during the middle six meters, averaging four to six steps per limb and having a testing time of less than five minutes.
- Two-minute walk test: a change in computed SSS and/or SRS of the lower limbs over time indicates the subject's ability to sustain gait quality over two minutes.
- Six-minute walk test: a change in computed SSS and/or SRS of the lower limbs over time at specific intervals (e.g., one, three, and five minutes) indicates the subject's ability to sustain gait quality over six minutes, as an endurance measure.

- Timed-Up and Go (TUG): SSS and/or SRS of the lower limbs can be computed in either direction as the subject stands, walks, turns, walks, and sits.
- Shoulder abduction/flexion test (SAT): SSS and/or SRS of the upper limbs can be computed as the subject performs shoulder abduction and/or flexion.
- Elbow range-of-motion test (ERT): SSS and/or SRS of the upper limbs can be computed as the subject performs elbow flexion and extension.

The disclosed symmetry and/or repeatability metrics may be used by clinicians to perform one or more of the following:

- Determine a subject's gait quality between lower limbs with a simple metric of percentage of symmetry (e.g., SSS).
- Determine when a subject's gait quality is improving (e.g., when SSS and/or SRS increases towards 100%).
- Determine when a subject's gait quality is deteriorating (e.g., when SSS and/or SRS decreases away from 100%).
- Determine a subject's ability to sustain gait symmetry and repeatability (e.g., during the two-minute walk test).
- Determine a subject's gait symmetry and repeatability endurance (e.g., during the six-minute walk test).
- Objectively document changes in specific limb segments, symmetry between limbs, repeatability between limbs, and the associated therapies that facilitated the changes.
- Objectively determine when asymmetries occur during the gait cycle of a subject. For example, the percentage of gait cycle (PoGC), which is a point in a single human gait cycle (e.g., stride) at which an event occurs, can be determined. Visualization of the difference between motion segments can enable a clinician to understand where a limitation may exist, which may in turn influence symmetry and/or repeatability.
- Objectively document and monitor changes over time for specific patient populations. For example, as the symmetry and/or repeatability metrics for a patient improve, rehabilitation therapies may be employed to progress the patient as quickly and safely as possible. As another example, restoration to a satisfactory value of the symmetry and/or repeatability metric can document and assist with the determination of when a return to activity is safe and appropriate and potentially reduce the risk of re-injury. As another example, the detection of a decline in the symmetry and/or repeatability metric may suggest a need for alternative therapies (e.g., a degenerative knee may require a TKA, a plateau in strength gains or functional improvement in a patient with a stroke may indicate the need for orthotic interventions, etc.). As another example, as a patient with a progressive diagnosis (e.g., ALS, rheumatoid arthritis, etc.) is monitored, a decline in the symmetry and/or repeatability metric may suggest the need for additional therapies or palliative care.
- Provide feedback to a patient based on changes in the patient's symmetry and/or repeatability metric over time. For example, elderly patients suffering from a loss of balance can monitor changes in their symmetry and/or repeatability metrics, over time, as they perform home exercise programs, in order to encourage exercise compliance. As another example, patients recovering from surgery can observe objective changes in their symmetry and/or repeatability metrics, so that those whose metric(s) improve can progress and those whose metric(s) decline can seek additional care. As another example, patients with chronic pain (e.g., lower back pain) can observe changes in their symmetry and/or repeatability metrics resulting from a walking program or other movement program. Feedback, comprising the symmetry and/or repeatability metric, can be combined with other feedback, such as audio feedback.

The disclosed symmetry and/or repeatability metrics may be used by clinicians to inform one or more of the following decisions:

- If a single limb has good repeatability and the contralateral limb has poor repeatability, the clinician may be prompted to focus on the limb with poor repeatability.
- If both contralateral limbs have poor repeatability, a UMN or systemic problem may be the cause, and the clinician may be prompted to perform more global treatments.
- If a specific limb segment has a consistently lower-than-expected angular velocity or movement pattern, the clinician may be prompted to administer more targeted treatments. For example, decreased angular velocity in the thigh after THA may prompt therapies to the hip flexors. As another example, decreased angular velocity in the shank after knee surgery may prompt targeted therapies for knee flexion or extension.
- If there are differences in angular velocities at different percentages of the gait cycle, the clinician may be prompted to perform therapies for gait training that target timing or for movement re-education.
- If the patient is unable to sustain the symmetry and/or repeatability metric during the two-minute walk test, this may indicate that the patient is having difficulty with coordination or balance, and the clinician may be prompted to perform gait training therapies that are designed to improve movement patterns and reduce extraneous movements that increase the effort of walking.

- If SSS declines over time during the six-minute walk test (e.g., the SSS at the three-minute and five-minute intervals are lower than the SSS at the one-minute interval), the clinician may be prompted to perform therapies that target muscular endurance.

In an embodiment, the application may comprise a user interface (e.g., a graphical user interface displayed on a display of a processing system 100, a speaker that produces an audio signal, and/or the like) that provides the value of symmetry and/or repeatability metric (e.g., SSS and/or SRS) for a patient, as well as one or more suggestions or recommendations of a next course of action (e.g., a specific therapy) for treating the patient. For example, the user interface may prompt the clinician according to any of the decision logic listed above.

To this end, specific values or ranges of the values of the symmetry and/or repeatability metrics may be associated with specific recommendations (e.g., about suggested therapies or other next courses of action). Thus, once the application, executing on a processing system 100, computes the symmetry and/or repeatability metric (e.g., SSS and/or SRS), the application may automatically retrieve one or more specific recommendations associated with the value of the computed symmetry and/or repeatability metric (e.g., from main memory 115 and/or secondary memory 120), and provide the retrieved recommendation(s) to the clinician via a graphical user interface displayed on a display of processing system 100 and/or audio output through a speaker of processing system 100.

In an embodiment, the ranges of values and associated recommendations may be different for different patient populations. In this case, the application may receive or retrieve (e.g., from a patient medical record) an indication of the patient population associated with a patient, and select the recommendation(s) associated with both the symmetry and/or repeatability metric for the patient and the indicated patient population.

In addition, each range of values may be associated with one of a plurality of different colors, so as to color code the different ranges of values (e.g., within the graphical user interface). In one particular implementation, a Rehab Progression Index (RPI) may be used to guide treatment decisions. Different scales of the RPI may be employed for different patient populations. The RPI may be a color-coding system that assigns colors and/or suggested therapies or other courses of action to different ranges of values of SSS and/or SRS. The table below indicates one example implementation of the RPI for the SSS of patients rehabilitating a limb injury:

| SSS (%) range | RPI | Suggested Rehab Progress |
|---|---|---|
| 80-100 | green | advanced skills, return to activity as appropriate |
| 50-79 | yellow | intermediate skills, limited return to activity, protection as appropriate |
| $\leq 49$ | red | protective skills, no activity |

In the above example, an RPI represented by the color green indicates that SSS is at or above 80%, and is associated with a recommendation that the patient may return to activities. An RPI represented by the color yellow indicates that SSS is within 50%-79%, and is associated with a recommendation that the patient may be ready to progress to an intermediate rehabilitation program. An RPI represented by the color red indicates that SSS is less than or equal to 49%, and is associated with a recommendation that the patient should not participate in strenuous activities and may require supervised rehabilitation practice or games. However, in general, the RPI should not be used as an absolute guide for a return to activity or rehabilitation progression, without additional measurement testing and professional interpretation of physical limitations and progress.

The invention is defined by the appended claims.

## Claims

1. A method comprising using at least one hardware processor (110) to:

   acquire gait data comprising a signal from each of a plurality of inertial sensors, wherein each signal represents an angular velocity of one of a plurality of lower limb segments of a subject during ambulation;
   segment each signal into a plurality of stride signals, wherein each of the plurality of stride signals represents one of a plurality of strides during the ambulation;
   calculate at least one gait metric based on the pluralities of stride signals, wherein the at least one gait metric comprises one or both of

      a gait symmetry metric that represents a similarity of the plurality of stride signals across two of the signals acquired for at least one pair of contralateral ones of the plurality of lower limb segments, and

a gait repeatability metric that represents a similarity of the plurality of stride signals within at least one of the signals to each other; and

output the at least one gait metric,
wherein the at least one gait metric comprises the gait symmetry metric, and wherein calculating the gait symmetry metric comprises aligning contralateral pairs of the plurality of stride signals across the two signals acquired for the at least one pair of contralateral lower limb segments,
**characterized in that**

- calculating the gait symmetry metric further comprises: calculating a distance between each aligned contralateral pair of stride signals; and calculating a mean of the calculated distances; and/or
- the distance is a Euclidean distance; and/or
- the gait symmetry metric is calculated as:

$$100 - \left( 100 \times \frac{mean\ of\ the\ calculated\ distances}{threshold_S} \right)$$

wherein $threshold_S$ is a threshold representing an estimated maximum possible mean of the calculated distances.

2. The method of Claim 1, wherein the signal is acquired for each of the plurality of lower limb segments of the subject during ambulation, and wherein the plurality of lower limb segments comprises a right thigh, right shank, left thigh, and left shank of the subject.

3. The method of Claim 1, wherein each signal represents the angular velocity in a sagittal plane of the subject over a time period of the ambulation.

4. The method of Claim 1, wherein acquiring the gait data comprises receiving a wireless signal transmitted by one or more inertial measurement units, positioned on the plurality of lower limb segments of the subject, wherein the one or more inertial measurement units comprise the plurality of inertial sensors.

5. The method of Claim 1, wherein the ambulation comprises one or more ambulation tests.

6. The method of Claim 5, wherein the one or more ambulation tests comprise a distance-based walk test.

7. The method of Claim 6, wherein the gait data consists of signals from the plurality of inertial sensors collected during a middle portion of the distance-based walk test.

8. The method of Claim 1, wherein each of the plurality of stride signals represents one of a plurality of strides from a toe-off to a next toe-off.

9. The method of Claim 1, wherein aligning the contralateral pairs of stride signals comprises dynamic time warping.

10. The method of Claim 1, wherein the at least one gait metric comprises the gait repeatability metric, and wherein calculating the gait repeatability metric comprises aligning the plurality of stride signals within the at least one signal.

11. The method of Claim 10, wherein aligning the plurality of stride signals within the at least one signal comprises dynamic time warping.

12. The method of Claim 10, wherein calculating the gait repeatability metric further comprises:

averaging the aligned plurality of stride signals within the at least one signal into an average stride signal;
calculating a distance between each of the plurality of stride signals within the at least one signal and the average stride signal; and
calculating a mean of the calculated distances.

13. The method of Claim 12, wherein the distance is a Euclidean distance.

**14.** The method of Claim 12, wherein the gait repeatability metric is calculated as:

$$100 - \left(100 \times \frac{mean\ of\ the\ calculated\ distances}{threshold_R}\right)$$

wherein *threshold$_R$* is a threshold representing an estimated maximum possible mean of the calculated distances.

**15.** A system (100) comprising:

at least one hardware processor (110); and
one or more software modules that are configured to, when executed by the at least one hardware processor (110), execute the method according to any one of the preceding claims.

**16.** A non-transitory computer-readable medium having instructions stored therein, wherein the instructions, when executed by a processor (110), cause the processor (110) to execute the method according to any one of the preceding method claims.


**Patentansprüche**

**1.** Verfahren, das die Verwendung mindestens eines Hardware-Prozessors (110) umfasst, um:

Gangdaten mit einem Signal von jedem von einer Vielzahl von Trägheitssensoren zu erfassen, wobei jedes Signal eine Winkelgeschwindigkeit von einem von einer Vielzahl von Segmenten von unteren Extremitäten eines Probanden während des Gehens darstellt;
jedes Signal in eine Vielzahl von Schrittsignalen zu segmentieren, wobei jedes der Vielzahl von Schrittsignalen einen von einer Vielzahl von Schritten während des Gehens darstellt;
mindestens eine Gangmetrik auf der Basis der Vielzahlen von Schrittsignalen zu berechnen, wobei die mindestens eine Gangmetrik eine oder beide umfasst von

einer Gangsymmetriemetrik, die eine Ähnlichkeit der Vielzahl von Schrittsignalen über zwei der Signale darstellt, die für mindestens ein Paar von kontralateralen der Vielzahl von Segmenten von unteren Extremitäten erfasst werden, und
eine Gangreproduzierbarkeitsmetrik, die eine Ähnlichkeit der Vielzahl von Schrittsignalen innerhalb mindestens eines der Signale zueinander darstellt; und

die mindestens eine Gangmetrik auszugeben,
wobei die mindestens eine Gangmetrik die Gangsymmetriemetrik umfasst, und wobei das Berechnen der Gangsymmetriemetrik das Justieren von kontralateralen Paaren der Vielzahl von Schrittsignalen über die zwei Signale, die für das mindestens eine Paar von kontralateralen Segmenten von unteren Extremitäten erfasst werden, umfasst,
**dadurch gekennzeichnet, dass**

- das Berechnen der Gangsymmetriemetrik ferner umfasst: Berechnen eines Abstandes zwischen jedem justierten kontralateralen Paar von Schrittsignalen und Berechnen eines Mittelwerts der berechneten Abstände; und/oder
- der Abstand ein euklidischer Abstand ist; und/oder
- die Gangsymmetriemetrik berechnet wird als:

$$100 - \left(100\ x\ \frac{Mittelwert\ der\ berechneten\ Abst\ddot{a}nde}{Schwellenwert_S}\right)$$

wobei *Schwellenwert$_S$* ein Schwellenwert ist, der einen abgeschätzten maximal möglichen Mittelwert der berechneten Abstände darstellt.

**2.** Verfahren nach Anspruch 1, wobei das Signal für jedes der Vielzahl von Segmenten von unteren Extremitäten des Probanden während des Gehens erfasst wird, und wobei die Vielzahl von Segmenten von unteren Extremitäten einen rechten Oberschenkel, einen rechten Unterschenkel, einen linken Oberschenkel und einen linken Unterschenkel des Probanden umfasst.

3. Verfahren nach Anspruch 1, wobei jedes Signal die Winkelgeschwindigkeit in einer Sagittalebene des Probanden über eine Zeitdauer des Gehens darstellt.

4. Verfahren nach Anspruch 1, wobei das Erfassen der Gangdaten das Empfangen eines drahtlosen Signals umfasst, das durch eine oder mehrere Trägheitsmesseinheiten übertragen wird, die an der Vielzahl von Segmenten von unteren Extremitäten des Probanden positioniert sind, wobei die eine oder die mehreren Trägheitsmesseinheiten die Vielzahl von Trägheitssensoren umfassen.

5. Verfahren nach Anspruch 1, wobei das Gehen einen oder mehrere Gehtests umfasst.

6. Verfahren nach Anspruch 5, wobei der eine oder die mehreren Gehtests einen distanzbasierten Lauftest umfassen.

7. Verfahren nach Anspruch 6, wobei die Gangdaten aus Signalen von der Vielzahl von Trägheitssensoren bestehen, die während eines mittleren Teils des distanzbasierten Lauftests gesammelt werden.

8. Verfahren nach Anspruch 1, wobei jedes der Vielzahl von Schrittsignalen einen von einer Vielzahl von Schritten von einem Abheben der Zehen bis zu einem nächsten Abheben der Zehen darstellt.

9. Verfahren nach Anspruch 1, wobei das Justieren der kontralateralen Paare von Schrittsignalen eine dynamische Zeitverschiebung umfasst.

10. Verfahren nach Anspruch 1, wobei die mindestens eine Gangmetrik die Gangreproduzierbarkeitsmetrik umfasst und wobei das Berechnen der Gangreproduzierbarkeitsmetrik das Justieren der Vielzahl von Schrittsignalen innerhalb des mindestens einen Signals umfasst.

11. Verfahren nach Anspruch 10, wobei das Justieren der Vielzahl von Schrittsignalen innerhalb des mindestens einen Signals eine dynamische Zeitverschiebung umfasst.

12. Verfahren nach Anspruch 10, wobei das Berechnen der Gangreproduzierbarkeitsmetrik ferner umfasst:

Mitteln der justierten Vielzahl von Schrittsignalen innerhalb des mindestens einen Signals zu einen mittleren Schrittsignal;
Berechnen eines Abstandes zwischen jedem der Vielzahl von Schrittsignalen innerhalb des mindestens einen Signals und dem mittleren Schrittsignal; und
Berechnen eines Mittelwerts der berechneten Abstände.

13. Verfahren nach Anspruch 12, wobei der Abstand ein euklidischer Abstand ist.

14. Verfahren nach Anspruch 12, wobei die Gangreproduzierbarkeitsmetrik berechnet wird als:

$$100 - \left( 100 \ x \ \frac{Mittelwert\ der\ berechneten\ Abst\ddot{a}nde}{Schwellenwert_R} \right)$$

wobei *Schwellenwert$_R$* ein Schwellenwert ist, der einen abgeschätzten maximal möglichen Mittelwert der berechneten Abstände darstellt.

15. System (100), das umfasst:

mindestens einen Hardware-Prozessor (110); und
ein oder mehrere Software-Module, die dazu eingerichtet sind, wenn sie durch den mindestens einen Hardware-Prozessor (110) ausgeführt werden, das Verfahren nach einem der vorangehenden Ansprüche auszuführen.

16. Nichttransitorisches computerlesbares Medium, in dem Anweisungen gespeichert sind, wobei die Anweisungen, wenn sie durch einen Prozessor (110) ausgeführt werden, bewirken, dass der Prozessor (110) das Verfahren nach einem der vorangehenden Verfahrensansprüche ausführt.

**Revendications**

1. Procédé comportant l'utilisation d'au moins un processeur matériel (110) pour :

   acquérir des données d'allure comportant un signal provenant de chaque capteur d'une pluralité de capteurs inertiels, chaque signal représentant une vitesse angulaire d'un segment d'une pluralité de segments de membre inférieur d'un sujet pendant une ambulation ;
   segmenter chaque signal en une pluralité de signaux de foulée, chaque signal de la pluralité de signaux de foulée représentant une foulée d'une pluralité de foulées pendant l'ambulation ;
   calculer au moins une grandeur métrique d'allure sur la base des pluralités de signaux de foulée, la au moins une grandeur métrique d'allure comportant un ou deux éléments parmi :

   une grandeur métrique de symétrie d'allure qui représente une similitude de la pluralité de signaux de foulée à travers deux des signaux acquis pour au moins une paire de segments controlatéraux de la pluralité de segments de membre inférieur, et
   une grandeur métrique de répétabilité d'allure qui représente une similitude des signaux de la pluralité de signaux de foulée dans au moins un des signaux les uns par rapport aux autres ; et
   générer la au moins une grandeur métrique d'allure,
   la au moins une grandeur métrique d'allure comportant la grandeur métrique de symétrie d'allure, et le calcul de la grandeur métrique de symétrie d'allure comportant l'alignement de paires controlatérales de la pluralité de signaux de foulée à travers les deux signaux acquis pour la au moins une paire de segments de membre inférieur controlatéraux,
   **caractérisé en ce que**

   - le calcul de la grandeur métrique de symétrie d'allure comporte en outre : calculer une distance entre chaque paire controlatérale alignée de signaux de foulée ; et calculer une moyenne des distances calculées ; et/ou
   - la distance est une distance euclidienne ; et/ou
   - la grandeur métrique de symétrie d'allure est calculée par :

$$100 - \left(100 \times \frac{moyenne\ des\ distances\ calcul\acute{e}es}{seuil_S}\right)$$

   $seuil_S$ étant un seuil représentant une moyenne possible maximale estimée des distances calculées.

2. Procédé selon la revendication 1, dans lequel le signal est acquis pour chaque segment de la pluralité de segments de membre inférieur du sujet pendant une ambulation, et la pluralité de segments de membre inférieur comportant une cuisse droite, une jambe droite, une cuisse gauche et une jambe gauche du sujet.

3. Procédé selon la revendication 1, dans lequel chaque signal représente la vitesse angulaire dans un plan sagittal du sujet sur une période de temps de l'ambulation.

4. Procédé selon la revendication 1, dans lequel l'acquisition des données d'allure comporte la réception d'un signal sans fil transmis par une ou plusieurs unités de mesure inertielle, positionnées sur la pluralité de segments de membre inférieur du sujet, l'unité ou les unités de mesure inertielle comportant la pluralité de capteurs inertiels.

5. Procédé selon la revendication 1, dans lequel l'ambulation comporte un ou plusieurs tests ambulatoires.

6. Procédé selon la revendication 5, dans lequel le ou les tests ambulatoires comportent un test de marche basé sur la distance.

7. Procédé selon la revendication 6, dans lequel les données d'allure consistent en des signaux provenant de la pluralité de capteurs inertiels collectés pendant une partie centrale du test de marche basé sur la distance.

8. Procédé selon la revendication 1, dans lequel chaque signal de la pluralité de signaux de foulée représente une foulée d'une pluralité de foulées d'un décollement d'orteil à un décollement d'orteil suivant.

9. Procédé selon la revendication 1, dans lequel l'alignement des paires controlatérales de signaux de foulée comporte un alignement temporel dynamique.

10. Procédé selon la revendication 1, dans lequel la au moins une grandeur métrique d'allure comporte la grandeur métrique de répétabilité d'allure, et le calcul de la grandeur métrique de répétabilité d'allure comportant l'alignement de la pluralité de signaux de foulée dans au moins un signal.

11. Procédé selon la revendication 10, dans lequel l'alignement de la pluralité de signaux de foulée dans le au moins un signal comporte un alignement temporel dynamique.

12. Procédé selon la revendication 10, dans lequel le calcul de la grandeur métrique de répétabilité d'allure comporte en outre :

> établir la moyenne de la pluralité alignée de signaux de foulée dans le au moins un signal de foulée dans un signal de foulée moyen ;
> calculer une distance entre chaque signal de la pluralité de signaux de foulée dans le au moins un signal et le signal de foulée moyen ; et
> calculer une moyenne des distances calculées.

13. Procédé selon la revendication 12, dans lequel la distance est une distance euclidienne.

14. Procédé selon la revendication 12, dans lequel la grandeur métrique de répétabilité d'allure est calculée par :

$$100 - \left( 100 \times \frac{moyenne\ des\ distances\ calculées}{seuil_R} \right)$$

$seuil_R$ étant un seuil représentant une moyenne possible maximale estimée des distances calculées.

15. Système (100) comportant :

> au moins un processeur matériel (110) ; et
> un ou plusieurs modules logiciels qui sont configurés pour, lorsqu'ils sont exécutés par le au moins un processeur matériel (110), exécuter le procédé selon l'une quelconque des revendications précédentes.

16. Support non transitoire lisible par ordinateur dans lequel sont stockées des instructions, dans lequel les instructions, lorsqu'elles sont exécutées par un processeur (110), amènent le processeur (110) à exécuter le procédé selon l'une quelconque des revendications de procédé précédentes.

**100**

**FIG. 1**

212A — 210A — 212B — 210B — 212A — 212B — 200

**FIG. 2**

FIG. 3

400

```
        ╭─────────╮
        │  START  │
        ╰─────────╯
             │
             ▼
┌───────────────────────────┐
│    Receive Motion Data     │
│            410             │
└───────────────────────────┘
             │
             ▼
┌───────────────────────────┐
│    Segment Motion Data     │
│            420             │
└───────────────────────────┘
             │
             ▼
┌───────────────────────────┐
│  Align Contralateral Strides │
│            430             │
└───────────────────────────┘
             │
             ▼
┌─────────────────────────────────────────┐
│ Calculate Difference between Contralateral Strides │
│                   440                    │
└─────────────────────────────────────────┘
             │
             ▼
┌───────────────────────────┐
│   Output Symmetry Metric   │
│            450             │
└───────────────────────────┘
             │
             ▼
        ╭─────────╮
        │   END   │
        ╰─────────╯
```

FIG. 4

500

```
          ┌─────────┐
          │  START  │
          └─────────┘
               │
               ▼
┌─────────────────────────────────────────────────┐
│            Receive Motion Data                   │
│                   510                            │
└─────────────────────────────────────────────────┘
               │
               ▼
┌─────────────────────────────────────────────────┐
│            Segment Motion Data                   │
│                   520                            │
└─────────────────────────────────────────────────┘
               │
               ▼
┌─────────────────────────────────────────────────┐
│            Align Ipsilateral Strides             │
│                   530                            │
└─────────────────────────────────────────────────┘
               │
               ▼
┌─────────────────────────────────────────────────┐
│           Average Ipsilateral Strides            │
│                   540                            │
└─────────────────────────────────────────────────┘
               │
               ▼
┌─────────────────────────────────────────────────┐
│ Calculate Difference between Ipsilateral Strides │
│                and Average                       │
│                   550                            │
└─────────────────────────────────────────────────┘
               │
               ▼
┌─────────────────────────────────────────────────┐
│           Output Repeatability Metric            │
│                   560                            │
└─────────────────────────────────────────────────┘
               │
               ▼
          ┌─────────┐
          │   END   │
          └─────────┘
```

# FIG. 5

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62896672
- US 2013074272 A1
- US 2013324333 A1
- US 2016184985 A1


**Non-patent literature cited in the description**

- **BARTH et al.** Stride segmentation during free walk movements using multi-dimensional subsequence dynamic time warping on inertial sensor data. *Sensors*, 2015, vol. 15 (3), 6419-40
- **KIM et al.** Exposure to an extreme environment comes at a sensorimotor cost. *NPJ Microgravity*, 2018, vol. 4, 1-8
- **KIM et al.** Using inertial sensors to quantify balance and gait performance during the tandem walking test. *Sensors*, 2019, vol. 19 (4)
- **ALLSEITS et al.** Characterizing the impact of sampling rate and filter design on the morphology of lower limb angular velocities. *IEEE Sensors Journal*, 2019
- **ALLSEITS et al.** The development and concurrent validity of a real-time algorithm for temporal gait analysis using inertial measurement units. *J. Biomech.*, 2017, vol. 55, 27-33
- **ALLSEITS et al.** A practical step length algorithm using lower limb angular velocities. *J. Biomech.*, 2018, vol. 66, 137-44
- **SHROUT et al.** Intraclass correlations: uses in assessing rater reliability. *Pscyhol. Bull.*, 1979, vol. 86 (2), 420-8
- **RAZALI et al.** Power comparisons of Shapiro-Wilk, Kolmogorov-Smirnov, Lilliefors and Anderson-Darling test. *J. Stat. Model Analytics*, 2011, vol. 2 (1), 21-33

- **SALARIAN et al.** Gait assessment in Parkinson's disease: toward an ambulatory system for long-term monitoring. *IEEE Trans. Biomed. Eng.*, 2004, vol. 51 (8), 1434-43
- **LEE et al.** Detection of spatio-temporal gait parameters by using wearable motion sensors. *Conf. Proc. IEEE Eng. Med. Biol. Soc.*, 2005, vol. 7, 6836-9
- **SHUMWAY-COOK et al.** Motor control: translating research into clinical practice. Lippincott Williams & Wilkens, 2017
- **JAEGERS et al.** Prosthetic gait of unilateral transfemoral amputees: a kinematic study. *Arch. Phys. Med. Rehabil.*, 1995, vol. 76 (8), 736-43
- **ESHRAGHI et al.** Pistoning assessment in lower limb prosthetic sockets. *Prosthet. Orthot. Int.*, 2012, vol. 36 (1), 15-24
- **GHOLIZADEH et al.** The evidence-base for elevated vacuum in lower limb prosthetics: literature review and professional feedback. *Clin. Biomech.*, 2016, vol. 37, 108-16
- **JAEGERS ; SEROUSSI et al.** Mechanical work adaptations of above-knee amputee ambulation. *Arch. Phys. Med. Rehabil.*, 1996, vol. 77 (11), 1209-14